(19) Europäisches Patentamt
European Patent Office
Office européen des brevets

(11) EP 4 209 229 A1

(12) **EUROPEAN PATENT APPLICATION**
published in accordance with Art. 153(4) EPC

(43) Date of publication:
**12.07.2023 Bulletin 2023/28**

(21) Application number: **21864421.9**

(22) Date of filing: **03.09.2021**

(51) International Patent Classification (IPC):
***A61K 47/54*** *(2017.01)* ***A61K 9/127*** *(2006.01)*
***A61K 45/00*** *(2006.01)* ***A61P 31/04*** *(2006.01)*

(52) Cooperative Patent Classification (CPC):
**A61K 9/127; A61K 45/00; A61K 47/54; A61P 31/04**

(86) International application number:
**PCT/JP2021/032378**

(87) International publication number:
**WO 2022/050369 (10.03.2022 Gazette 2022/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.09.2020 JP 2020148632**

(71) Applicants:
- **OSAKA UNIVERSITY**
  **Suita-shi**
  **Osaka 565-0871 (JP)**
- **Shionogi & Co., Ltd**
  **Osaka-shi, Osaka 541-0045 (JP)**

(72) Inventors:
- **MATSUZAKI Takashi**
  **Suita-shi, Osaka 565-0871 (JP)**
- **MAKI Hideki**
  **Toyonaka-shi, Osaka 561-0825 (JP)**
- **YOSHIDA Osamu**
  **Toyonaka-shi, Osaka 561-0825 (JP)**
- **HOMMA Tomoyuki**
  **Toyonaka-shi, Osaka 561-0825 (JP)**

(74) Representative: **Boult Wade Tennant LLP**
**Salisbury Square House**
**8 Salisbury Square**
**London EC4Y 8AP (GB)**

# (54) LIPOSOME PREPARATION CONTAINING ANTIBACTERIAL DRUG

(57) By administering a liposome formulation in which an antibacterial agent is bound to a liposome, in particular, a lipid-soluble side chain of the antibacterial agent is bound to a lipid of the liposome, and the antibacterial agent extends outward from a surface of the liposome, it is possible to provide a liposome formulation in which blood retention of an active ingredient is increased, an amount of the active ingredient taken by a reticuloendothelial system such as a liver is reduced, an amount of the active ingredient transferred into a kidney is reduced, and antibacterial activity can also be increased with little resistance.

Fig. 1

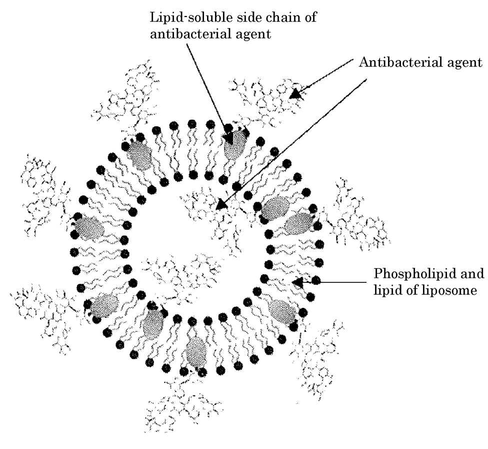


EP 4 209 229 A1

## Description

[TECHNICAL FIELD]

The present invention relates to a liposome formulation containing an antibacterial agent. Specifically, the present invention relates to a liposome formulation in which an antibacterial agent is bound outward to a surface layer of a liposome. In a liposome formulation in which an antibacterial agent is bound outward to a surface layer of a liposome, the liposome is less likely to be taken by a reticuloendothelial system, toxicity is reduced, blood retention can be maintained, and an antimicrobial action can be enhanced.

[BACKGROUND ART]

In recent years, development of antibacterial agents has been remarkable and has greatly contributed to treatment of infectious diseases. A first target of these antibacterial agents is an infectious causative bacterium. Therefore, antibacterial activity of a drug is important.

Among the antibacterial agents, glycopeptide antibiotics are antibiotics having complex polycyclic peptide structures produced by a variety of microorganisms, and provide antibacterial agents effective against most Gram-positive bacteria. In recent years, bacteria resistant to penicillins, cephalosporins, and the like have emerged, and infection by multidrug-resistant bacteria and methicillin-resistant Staphylococcus aureus (MRSA) has caused important problems in medical practice. Glycopeptide antibiotics like vancomycin are effective against such microorganisms, and particularly vancomycin has become a last tool drug for infection by MRSA and other resistant bacteria. However, as a side effect of the glycopeptide antibiotics, renal disorder is well known.

Amphotericin B has been used in injections as a therapeutic agent for deep-seated mycosis, but it is known to cause side effects such as renal disorder. Therefore, a liposome formulation in which a complex of amphotericin B and cholesterol is incorporated into a liposome membrane was found, and the liposome formulation had reduced nephrotoxicity, specifically acted on fungi, and exhibited antifungal activity. However, the liposome formulation is not a liposome formulation in which amphotericin B is bound outward to a surface layer of a liposome, disadvantages derived from the liposome have not been improved.

A liposome contains mainly a lipid derived from a living body as a component, and has higher safety than that of other drug delivery systems (DDSs). However, when the liposome is intravenously administered, there is a disadvantage that the liposome is taken by a reticuloendothelial system (RES) such as a liver or a spleen at a considerable rate.

As a liposome in which uptake of the liposome by the reticuloendothelial system is suppressed, a PEG-liposome in which an outer surface of the liposome is modified with polyethylene glycol (PEG) has been developed. This liposome avoids uptake by the reticuloendothelial system and can be retained in blood for a long time (Non-patent Document 1). However, most of active ingredients in the liposome are often encapsulated in the liposome, and the encapsulated active ingredients are not necessarily released from the liposome, and it is not clear whether or not activity of the active ingredients, particularly antibacterial activity for an antibacterial agent occurs efficiently.

Patent Document 1 discloses a liposome formulation encapsulating polymeric microparticles. An antibody or polyethylene glycol is bound outward to a surface layer of the liposome. However, it is not a liposome formulation in which an active ingredient of a pharmaceutical product is bound outward to a surface layer of the liposome.

Patent Document 2 discloses an immunostimulatory spherical nucleic acid (IS-SNA) containing a core having an oligonucleotide shell composed of an immunostimulatory oligonucleotide located on an outer surface of the core and a checkpoint inhibitor, in which the core is a liposome-based core. However, the liposome of the patent document does not contain an antibacterial agent.

Non-patent Document 2 discloses a nanoliposome containing an antibacterial agent and various phospholipids. However, it is not a liposome formulation in which an antibacterial agent as an active ingredient is bound outward to a surface layer of the liposome.

[PRIOR ART REFERENCES]

[Patent Document]

[Patent Document 1] JP 2008-512350 A
[Patent Document 2] JP 2019-514985 A

[Non-patent Document]

[Non-patent Document 1] Drug delivery system: controlled release absorption improvement drug targeting, 2nd edition, p. 124, supervised by Mitsuru Hashida, edited by Yoshinobu Takakura.
[Non-patent Document 2] Nanoliposome-based antibacterial drug delivery

[SUMMARY OF THE INVENTION]

[PROBLEMS TO BE SOLVED BY THE INVENTION]

There has been a demand for development of a liposome formulation that reduces side effects of antibacterial agents, particularly glycopeptide antibacterial agents, and cyclic lipopeptide antibacterial agents, and that is less resistant and generates antibacterial activity.

[MEANS FOR SOLVING THE PROBLEM]

As a result of intensive studies, the present inventors have found a liposome formulation in which an antibacterial agent is encapsulated in a liposome and the antibacterial agent is bound outward to a surface layer of the liposome, as shown in Fig. 1. The present inventors have found that in a liposome formulation in which a glycopeptide antibacterial agent or a cyclic lipopeptide antibacterial agent is bound outward to a surface layer of a liposome, an effect is maintained by increasing blood retention of an active ingredient, an amount of the active ingredient taken by a reticuloendothelial system such as a liver is reduced, and an amount of the active ingredient transferred into a kidney is reduced, so that side effects such as toxicity are reduced, and antibacterial activity can be generated with little resistance.
That is, the present invention relates to:

(1A) A liposome formulation, wherein an antibacterial agent is bound outward to a surface layer of a liposome.
(2A) The liposome formulation according to (1A), wherein a lipid-soluble side chain of the antibacterial agent is bound to a lipid of the liposome.
(3A) The liposome formulation according to (2A), wherein the lipid-soluble side chain of the antibacterial agent is a group represented by the following formulas:

[Chemical Formula 1]

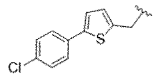 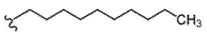 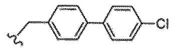

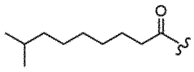 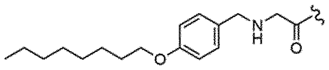

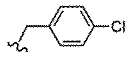 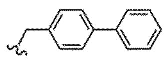

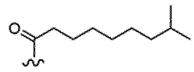 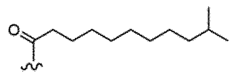

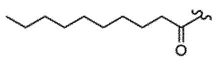 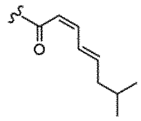

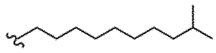 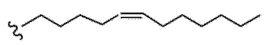

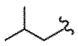 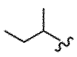

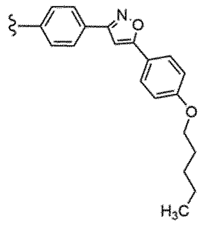 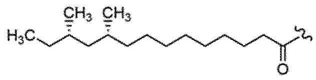

[Chemical Formula 2]

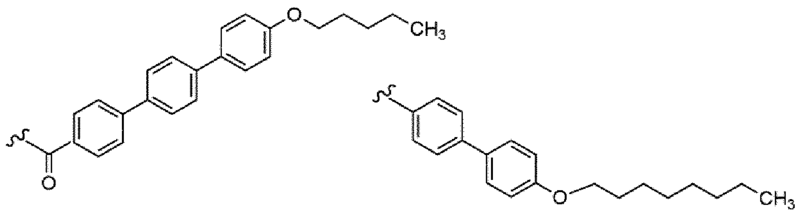

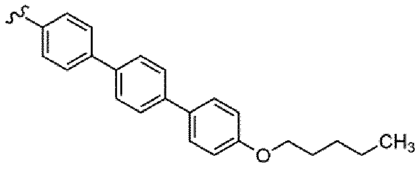

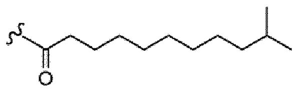

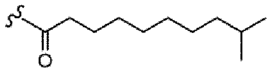

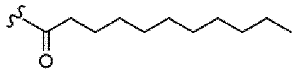

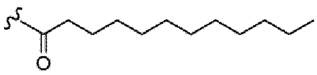

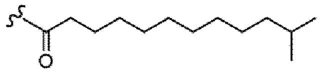

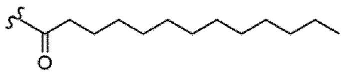

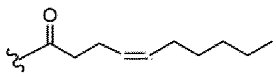

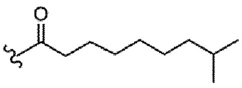

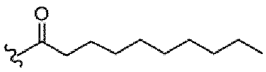

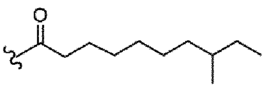

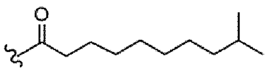

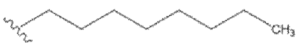

(4A) The liposome formulation according to any one of (1A) to (3A), wherein the antibacterial agent is a glycopeptide antibacterial agent or a cyclic lipopeptide antibacterial agent.
(5A) The liposome formulation according to (1A) or (2A), wherein the antibacterial agent is one or more selected from the group consisting of a compound represented by Formula (I):

[Chemical Formula 3]

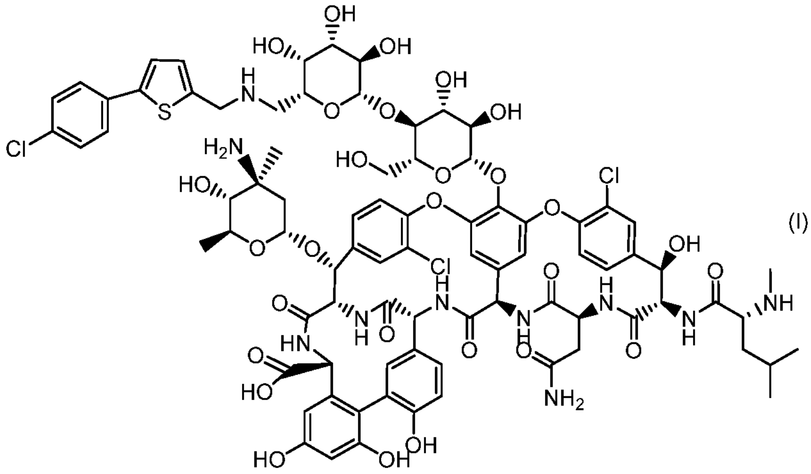

(I)

or a pharmaceutically acceptable salt thereof, telavancin, oritavancin, teicoplanin, dalbavancin, mideplanin, N'-p-octyloxybenzylglycyl-vancomycin, (4"R)-22-O-(3-amino-3-C-methyl-2,3,6-trideoxy-α-L-arabino-hexopyranosyl)-3"-[(4-chlorobenzyl)amino]-3"-deaminovancomycin, (4"R)-3"-N-(1, 1'-biphenyl-4-ylmethyl)-22-O-(3-amino-3-C-methyl-2,3,6-trideoxy-α-L-arabino-hexopyranosyl)vancomycin, and 5,31-dichloro-38-de(methoxycarbonyl)-7-demethyl-15-deamino-19-deoxy-56-O-[2-deoxy-2-[(10-methyl-1-oxoundecyl)amino]-β-D-glueopyranosyl]-38-[[[3-(dimethylamino)propyl]amino]carbonyl]-42-O-α-D-mannopyranosyl-15-(methylamino)ristomycin A aglycone, and YV11455.

(6A) The liposome formulation according to (1A) or (2A), wherein the antibacterial agent is oritavancin.

(7A) The liposome formulation according to any one of (1A) to (6A), wherein the liposome is comprised of one or two or more lipids.

(8A) The liposome formulation according to (7A), wherein the lipid constituting the liposome is one or more selected from the group consisting of a phospholipid, an ether glycerophospholipid, a sphingophospholipid, a glyceroglycolipid, a glycosphingolipid, a glyceride lipid, cardiolipin, a galactolipid, a mannolipid, galactolecithin, and cholesterol.

(9A) The liposome formulation according to (7A), wherein the lipid constituting the liposome is one or more selected from the group consisting of egg yolk lecithin, soybean lecithin, hydrogenated egg yolk lecithin, hydrogenated soybean lecithin, a hydrogenated soybean phospholipid (HSPC), soybean-derived phosphatidylcholine, soybean-derived hydrogenated phosphatidylcholine, a purified hydrogenated soybean phospholipid, phosphatidylcholine, egg yolk phosphatidylcholine, hydrogenated soybean phosphatidylcholine, dimyrisylphosphatidylcholine, dipalmitoylphosphatidylcholine, palmitoylstearoylphosphatidylcholine, dioleoylphosphatidylcholine, distearoylphosphatidylcholine, 1,2-dioleoyl-sn-glycero-3-phosphatidylcholine, distearylphosphatidylcholine, 1-palmitoyl-2-oleoyl-sn-glycero-3-phosphatidylcholine, phosphatidylethanolamine, soybean phosphatidylethanolamine, dimyristoylphosphatidylethanolamine, dipalmitoylphosphatidylethanolamine, dioleoylphosphatidylethanolamine, egg yolk phosphatidylethanolamine, monooleoylphosphatidylethanolamine, polyethylene glycol-distearoylphosphatidylethanolamine, phosphatidylglycerol, soybean phosphatidyl glycerol, dimyristoylphosphatidylglycerol, dipalmitoylphosphatidyl glycerol, dioleoylphosphatidyl glycerol, distearylphosphatidylglycerol, dielaidoylphosphatidylglycerol, palmitoylstearoylphosphatidylglycerol, egg yolk phosphatidylglycerol, phosphatidylserine, egg yolk phosphatidylserine, soybean phosphatidylserine, dimyristoylphosphatidylserine, dipalmitoylphosphatidylserine, dioleoylphosphatidylserine, dielaidoylphosphatidylserine, 1,2-dioleoyl-sn-glycero-3-phosphatidylserine, distearoylphosphatidylserine, phosphatidic acid, egg yolk phosphatidic acid, soybean phosphatidic acid, dimyristoylphosphatidic acid, dipalmitoylphosphatidic acid, dioleoylphosphatidic acid, distearylphosphatidic acid, dielaidoylphosphatidylamine, soybean-derived hydrogenated phosphatidylinositol, egg yolk phosphatidylinositol, soybean phosphatidylinositol, dipalmitoylphosphatidylinositol, 1,2-dioleoyl-sn-glycero-3-phosphatidylinositol, dimyristoylphosphatidylinositol, distearoylphosphatidylinositol, cholesterol, cardiolipin, sphingomyelin, hexadecylphosphocholine, N-[1-(2,3-dioleyloxy)propyl]-N,N,N-trimethylammonium chloride, 1,2-bis(oleoyloxy)-3-(trimethylammonio)propane, 1,2-bis(hexadecyloxy)-3-trimethylaminopropane, 3[beta][N-(N'N'-dimethylaminoethane)-carbamyl] cholesterol, 1,2-dimyristroyl-sn-glycero-3-phosphocholine, 1,2-dilauroyl-sn-glycero-3-phosphocholine, 1,2-distearoyl-sn-glycero-3-phosphocholine, 1,2-dimyristoyl-sn-glycero-3-phosphoethanolamine, 1,2-dipalmitoyl-sn-glycero-3-phosphoethanolamine, 1,2-dioleoyl-sn-glycero-3-phosphate monosodium salt, 1,2-dipalmitoyl-sn-glycero-3-[phosphor-rac-(l-glycerol)] sodium salt, 1,2-dimyristoyl-sn-glycero-3-[phospho-L-serine] sodium salt, 1,2-dioleoyl-sn-glycero-3-phosphoethanolamine-N-glutaryl sodium salt, 1,1',2,2'-tetramyristoyl cardiolipin ammonium salt, a glyceride lipid, cardiolipin, a galactolipid, a mannolipid, and galactolecithin.

(10A) The liposome formulation according to (7A), wherein the lipid constituting the liposome is a hydrogenated soybean phospholipid (HSPC) and/or cholesterol.

(11A) The liposome formulation according to (1A) or (2A), wherein the antibacterial agent is oritavancin and the lipid constituting the liposome is a hydrogenated soybean phospholipid (HSPC) and cholesterol.

(12A) The liposome formulation according to any one of (1A) to (11A), wherein a surface charge of the liposome is a positive charge.

(13A) The liposome formulation according to any one of (1A) to (12A), wherein an average particle size of the liposome is 30 to 120 nm.

(14A) The liposome formulation according to any one of (1A) to (13A), wherein a weight ratio of the lipid constituting the liposome to the antibacterial agent in the liposome is 15 : 1 to 20 : 1.

(15A) A method for producing a liposome formulation in which an antibacterial agent is bound outward to a surface layer of a liposome, the method comprising the steps of:

i) mixing a first solution in which one or more lipids are dissolved in a first organic solvent and a second solution in which an antibacterial agent is dissolved in a second organic solvent to obtain a third solution;
ii) providing one or more first flows of the third solution, a feed rate of which is a linear velocity of 1.0 m/min or more;
iii) providing one or more second flows of the first aqueous solution, a feed rate of which is a linear velocity of 1.1 m/min or more;
iv) combining the one or more first flows and the one or more second flows at a first crossing point to mix components of the first and second flows and providing a first combined flow containing the antibacterial agent-containing liposome; and
v) in a first direction, flowing the first combined flow and providing an outlet solution containing the antibacterial agent-containing liposome.

(16A) The production method according to (15A), wherein the first and second organic solvents are miscible with the first aqueous solution, either the first and second organic solvents or the first aqueous solution contains a buffer, and a concentration of the first organic solvent in the outlet solution is less than 45%.

(17A) The production method according to (15A) or (16A), wherein the first organic solvent contains a lower alkanol solvent, the second organic solvent contains an aprotic polar solvent, and the buffer is an aqueous solution containing a basic peptide and/or an alkali metal salt.

(18A) The production method according to (17A), wherein the lower alkanol solvent comprises ethanol, the aprotic polar solvent comprises dimethyl sulfoxide, and the buffer comprises histidine and/or sodium phosphate.

(19A) The production method according to any one of (15A) to (18A), further comprising a step of incubating the outlet solution.

(20A) The production method according to (19A), further comprising a step of concentrating and dialyzing the incubated outlet solution.

(21A) The production method according to any one of (15A) to (20A), wherein about 80% or more of the antibacterial agent is encapsulated in a lipid nanoparticle in the outlet solution.

[EFFECT OF THE INVENTION]

As shown in Fig. 1, the liposome formulation containing an antibacterial agent of the present invention is a liposome formulation in which an antibacterial agent is bound outward to a surface layer of a liposome, and can reduce side effects and generate antibacterial activity with little resistance. Outward binding of the antibacterial agent to the surface layer of the liposome can improve disadvantages of the liposome.

[BRIEF DESCRIPTION OF THE DRAWINGS]

Fig. 1 is a schematic view of a liposome formulation of the present invention.

Fig. 2 shows changes in plasma concentration after administration of a telavancin-containing liposome (Examples 4 and 5).

Fig. 3 shows changes in plasma concentration after administration of an oritavancin-containing liposome (Example 1, Examples 6 to 8).

[MODE FOR CARRYING OUT THE INVENTION]

The meaning of each term used in the present description will be described below. Unless otherwise specified, each term is used in the same meaning when used alone or in combination with another term.

The term "consisting of" means having only components.

The term "including" means being not limited to the components, but not excluding elements that are not described.

Hereinafter, the present invention will be described with reference to embodiments. It should be understood that throughout the present description, a singular form of expression includes the concept of its plural form, unless otherwise specified. Accordingly, it should be understood that an article in singular form (for example, in English, "a", "an", "the", and the like) includes the concept of its plural form, unless otherwise specified.

Furthermore, it should be understood that the terms used in the present description are used in the meaning commonly used in the art, unless otherwise specified. Therefore, unless otherwise defined, all technical and scientific terms used in the present description have the same meanings as those commonly understood by those skilled in the art to which the present invention belongs. In case of conflict, the present description (including definitions) prevails.

The present invention is a liposome formulation in which an antibacterial agent is bound outward to a surface layer of a liposome. In the liposome formulation, the antibacterial agent may be bound outward to the surface layer of the liposome, further the liposome may encapsulate the antibacterial agent, and the antibacterial agent may also be bound inward to an inner layer of the liposome.

When the antibacterial agent is bound outward to the surface layer of the liposome, a liposome formulation in which a lipid-soluble side chain of the antibacterial agent is bound to a lipid of the liposome by a hydrophobic interaction is also included. The lipid-soluble side chain of the antibacterial agent also includes a lipid-soluble side chain of the antibacterial agent itself, and also includes a lipid-soluble side chain introduced by applying chemical modification to the antibacterial agent.

The term "antibacterial agent" as used herein means a compound exhibiting antibacterial activity, which is a drug substance, namely an active ingredient of a pharmaceutical product. The term "antifungal agent" as used herein means a compound exhibiting antifungal activity, which is a drug substance, namely an active ingredient of a pharmaceutical product.

The term "liposome" as used herein refers to a closed vesicle formed by a lipid bilayer membrane, and exists in a state of a suspension in which an internal aqueous phase in a closed space and an external aqueous phase exist across the membrane. The lipid bilayer membrane is preferably a phospholipid bilayer membrane.

As a membrane structure of the liposome, a unilamellar vesicle consisting of a single layer of the lipid bilayer membrane and a multilamellar vesicle (MLV), and a small unilamellar vesicle (SUV) and a large unilamellar vesicle (LUV) as unilamellar vesicles are known. That is, the liposome may be a monolayer consisting of a single bilayer, or a multilayer consisting of two or more concentric bilayers. A bilayer of a phospholipid is formed from two layers of phospholipid molecules. In the present invention, the membrane structure is not particularly limited.

A liposome containing a phospholipid, an ether glycerophospholipid, a sphingophospholipid, a glyceroglycolipid, and a glycosphingolipid as main components of the liposome membrane, and further containing sterols, tocopherols, and the like as lipid components for stabilizing the liposome membrane, or the like is preferably used.

A phospholipid is a molecule that has two major regions, a hydrophilic head region containing a phosphate of an organic molecule and a tail of one or more hydrophobic fatty acids. In particular, a naturally occurring phospholipid has a hydrophilic region containing choline, glycerol, and a phosphate, and two hydrophobic regions containing fatty acids. When the phospholipid is placed in an aqueous environment, the hydrophilic heads gather in a linear configuration, and these hydrophobic tails essentially align in parallel with one another. The second line of the molecule then aligns with the first line at each tail, as the hydrophobic tails try to avoid the aqueous environment. In order to maximally avoid contact with the aqueous environment (that is, at an edge of a bilayer), and at the same time to minimize a surface area to volume ratio and thereby achieve a minimum energy configuration, two lines of the phospholipid, known as a phospholipid bilayer or a lamella, converge spherically, capturing whatever can be dissolved or suspended in a core of the sphere, such as some aqueous vehicles and an active ingredient of a pharmaceutical product.

With respect to a liposomal component of a pharmaceutical formulation of the present invention, the term "liposome" means any lipid composition that can be used to deliver a compound, in which an aqueous volume is encapsulated by an amphiphilic lipid bilayer, or an interior containing an antibacterial agent or a combination of antibacterial agents is coated with a lipid, and in the formulation of the present invention, the antibacterial agent is bound to a surface layer of the liposome via a lipid-soluble side chain, and the antibacterial agent faces outward.

The active ingredient used in the present invention is an antibacterial agent or an antifungal agent of a penicillin type, a cephem type, a carbapenem type, an oxacephem type, an aminoglycoside type, a macrolide type, a tetracycline type, a chloramphenicol type, an oxazolidinone type, a clindamycin type, a quinolone type, a sulfa agent, a trimethoprim type, a fosfomycin type, isoniazid, a rifamycin type, pyrazinamide, ethambutol, delamanid, pretomanid, a polyene macrolide type, an azole type, or an allylamine type. Specifically, the active ingredient is a highly toxic glycopeptide antibacterial agent, a cyclic lipopeptide antibacterial agent, an echinocandin antifungal agent, or the like. The antibacterial agent is preferably a glycopeptide antibacterial agent or a cyclic lipopeptide antibacterial agent, and more preferably a glycopeptide antibacterial agent.

With respect to the antibacterial agent of the pharmaceutical formulation of the present invention, the antibacterial agent of the present invention may exert a bactericidal or bacteriostatic activity. In various embodiments of the present invention, the pharmaceutical formulation can contain two or more antibacterial agents, for example, the formulation can contain a combination of bactericidal and bacteriostatic antibacterial agents. Generally, exerting bactericidal activity against bacteria means that the antibacterial agent has ability of the composition to kill or critically damage one or more species of bacteria that are susceptible to an antibiotic of the composition. However, an antibacterial agent having bacteriostatic activity has ability to inhibit growth of one or more species of bacteria without killing one or more species of target bacteria that are susceptible to the antibiotic of the composition. In a preferred embodiment of the present invention, the antibacterial agent of the pharmaceutical formulation is bactericidal or bacteriostatic against Gram-positive bacteria, including, but not limited to, methicillin-resistant Staphylococcus aureus (MRSA) bacteria. In various embodiments of the present invention, the antibacterial agent that is the active ingredient of the present invention is a glycopeptide antibacterial agent (glycopeptide antibiotic). The term "glycopeptide antibacterial agent" as used in the context of the present invention and known to those skilled in the art means an antibacterial agent having a mechanism of action including inhibiting growth of a bacterial cell wall. Antibacterial agents in this class of the glycopeptide antibacterial agent are not limited thereto. Examples thereof include (1S,2R,18R,19R,22S,25R,28R,40S)-2-(3-amino-2,3,6-trideoxy-3-C-methyl-α-L-arabino-hexopyranosyloxy)-22-carbamoylmethyl-5,15-dichloro-50-(6-{[5-(4-chlorophenyl)thiophen-2-yl]methylamino}-6-deoxy-5-D-galactopyranosyl-(1→4)-β-D-glucopyranosyloxy) -18, 32, 35, 37-tetrahydroxy-19- [(2R)-4-methyl- 2-(methylamino)pentanoylamino]-20,23,26,42,44-pentaoxo-7,13-dioxa-21,24,27,41,43-pentaazaoctacyclo [26.14.2.23,6.214,17.18,12.129,33.010,25.034,39]pentaconta-3,5,8,10,12(50),14,16,29,31,33(49),34,36,38,45,47-pentadecaene-40-carboxylic acid (hereinafter, the present compound may be referred to as "compound A"), telavancin, oritavancin, teicoplanin (Gruppro Lepetit), dalbavancin (BI-397, Pfizer Inc), mideplanin (MDL62873, Marion Merrell Dow), N'-p-octyloxybenzylglycyl-vancomycin (GINA-220, Russian Academy & Chiron), (4"R)-22-O-(3-amino-3-C-methyl-2,3,6-trideoxy-α-L-arabino-hexopyranosyl)-3"-[(4-chlorobenzyl)amino]-3"-deaminovancomycin, (4"R)-3"-N-(1,1'-biphenyl-4-ylmethyl)-22-O-(3-amino-3-C-methyl-2,3,6-trideoxy-α-L-arabino-hexopyranosyl)vancomycin, 5,31-dichloro-38-de(methoxycarbonyl)-7-demethyl-15-deamino-19-deoxy-56-O-[2-deoxy-2-[(10-methyl-1-oxoundecyl)amino]-β-D-glucopyranosyl]-38-[[[3-(dimethylamino)propyl]amino]carbonyl]-42-O-α-D-mannopyranosyl-15-(methylamino)ristomycin A aglycone, LY191145/LY307599 (Eli Lilly and Company), and MDL63246/MDL63042 (Marion Merrell Dow), YV11415, and derivatives thereof. The antibacterial agent is preferably compound A, telavancin, or oritavancin, and more preferably oritavancin.

Compound A
[Chemical Formula 4]

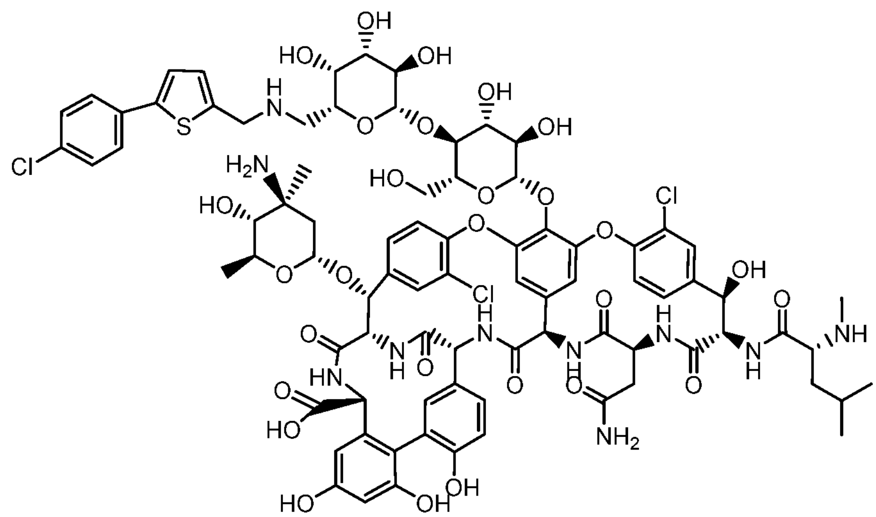

Telavancin

[Chemical Formula 5]

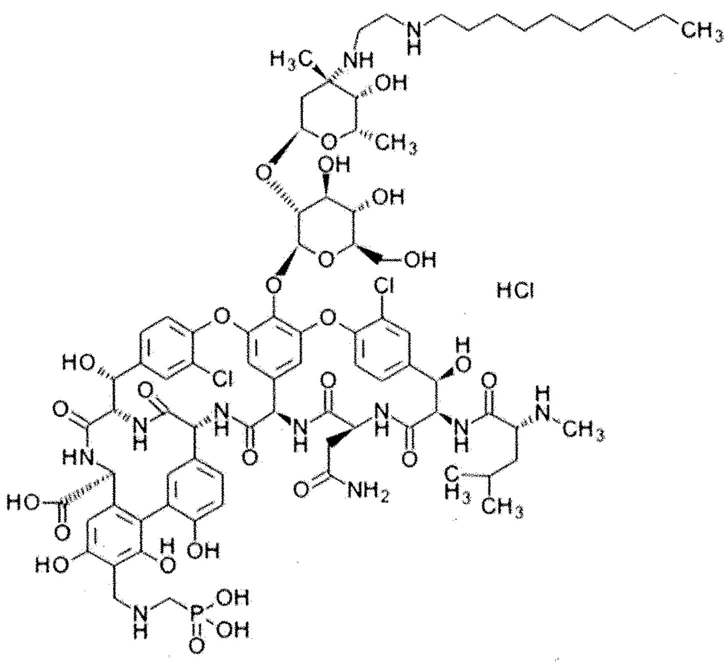

Oritavancin

[Chemical Formula 6]

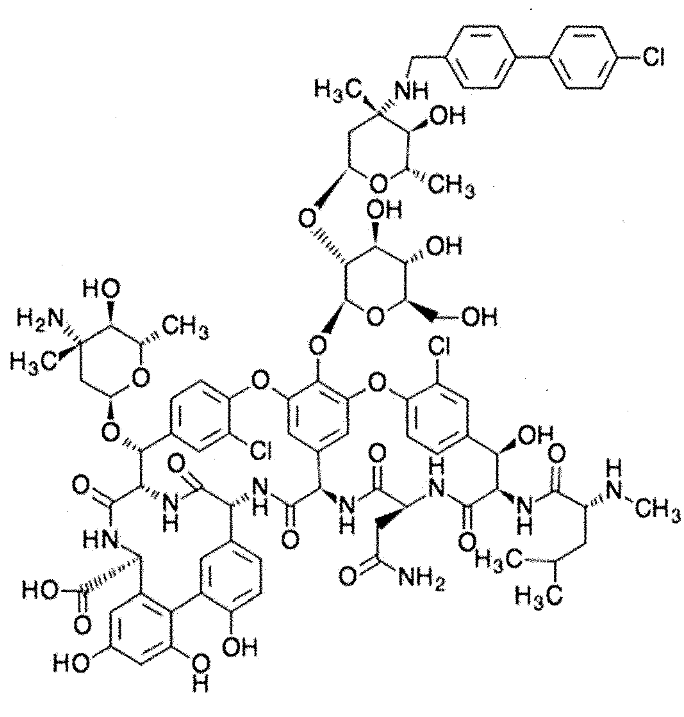

Teicoplanin $A_{2-2}$

[Chemical Formula 8]

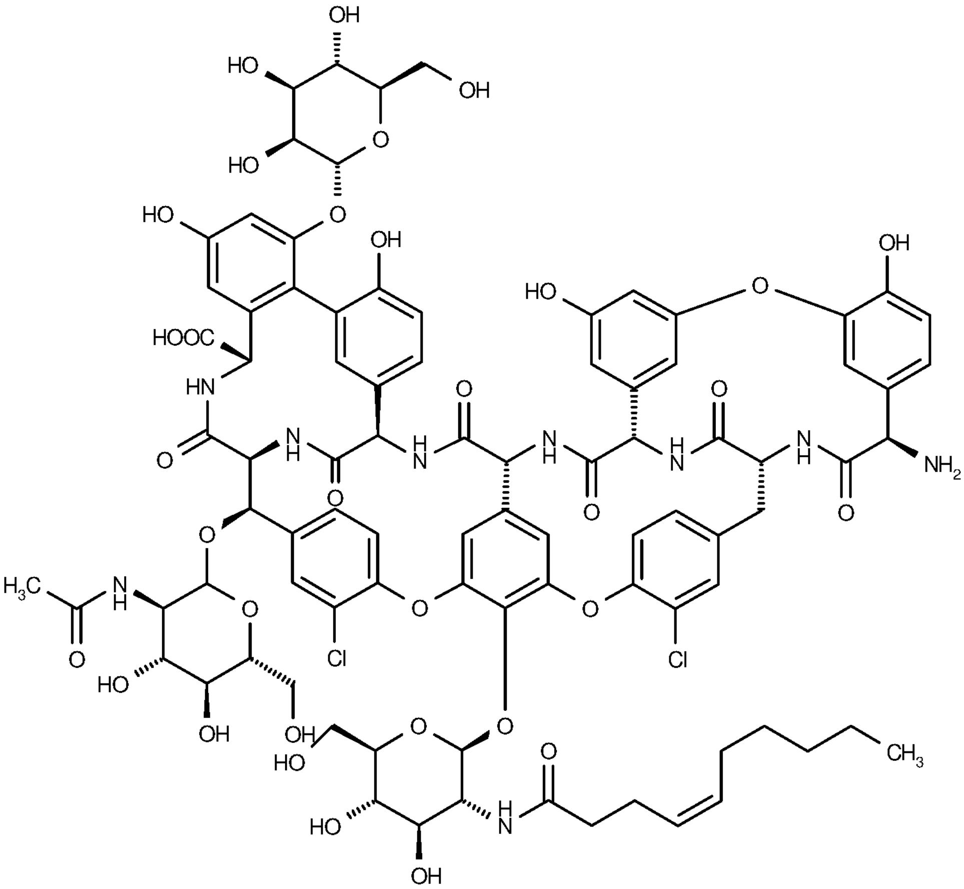

teicoplanin $A_{2-1}$

Teicoplanin $A_{2-3}$

[Chemical Formula 7]

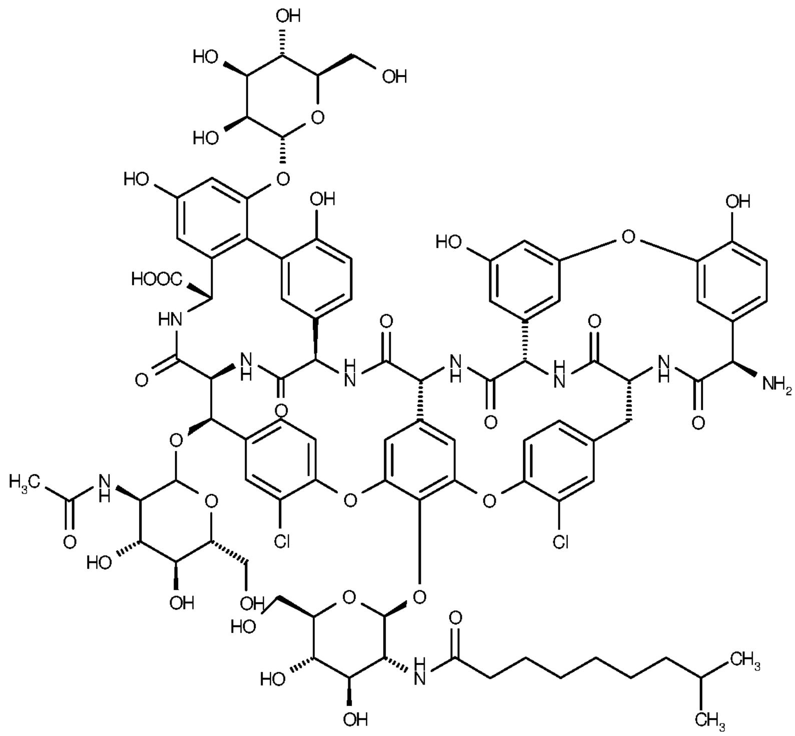

Teicoplanin $A_{2-1}$

[Chemical Formula 9]

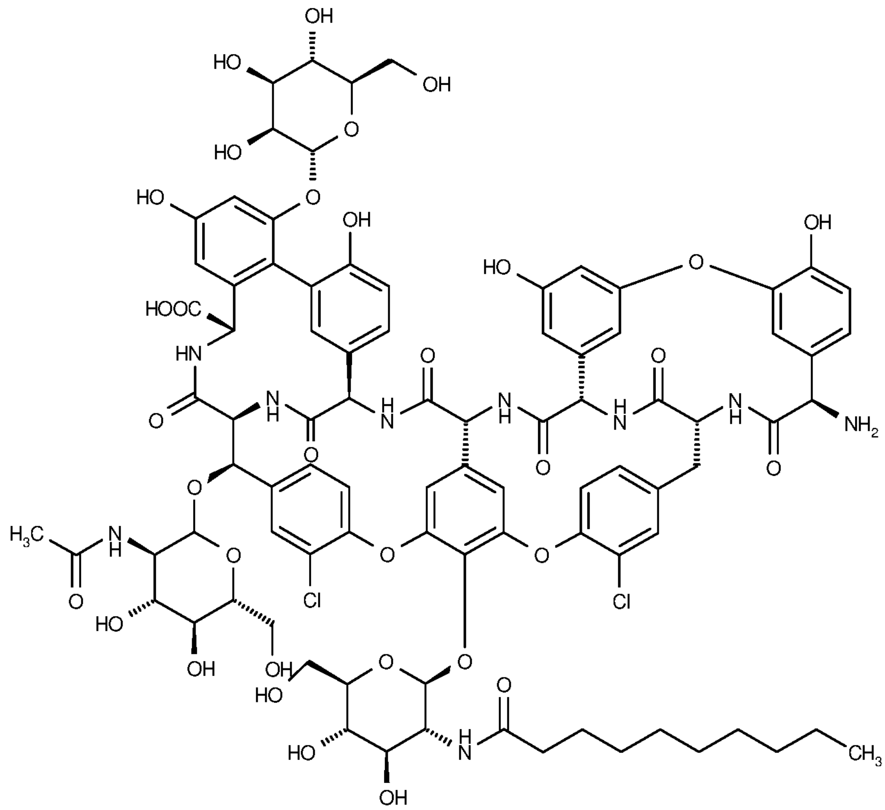

Teicoplanin $A_{2-4}$

[Chemical Formula 10]

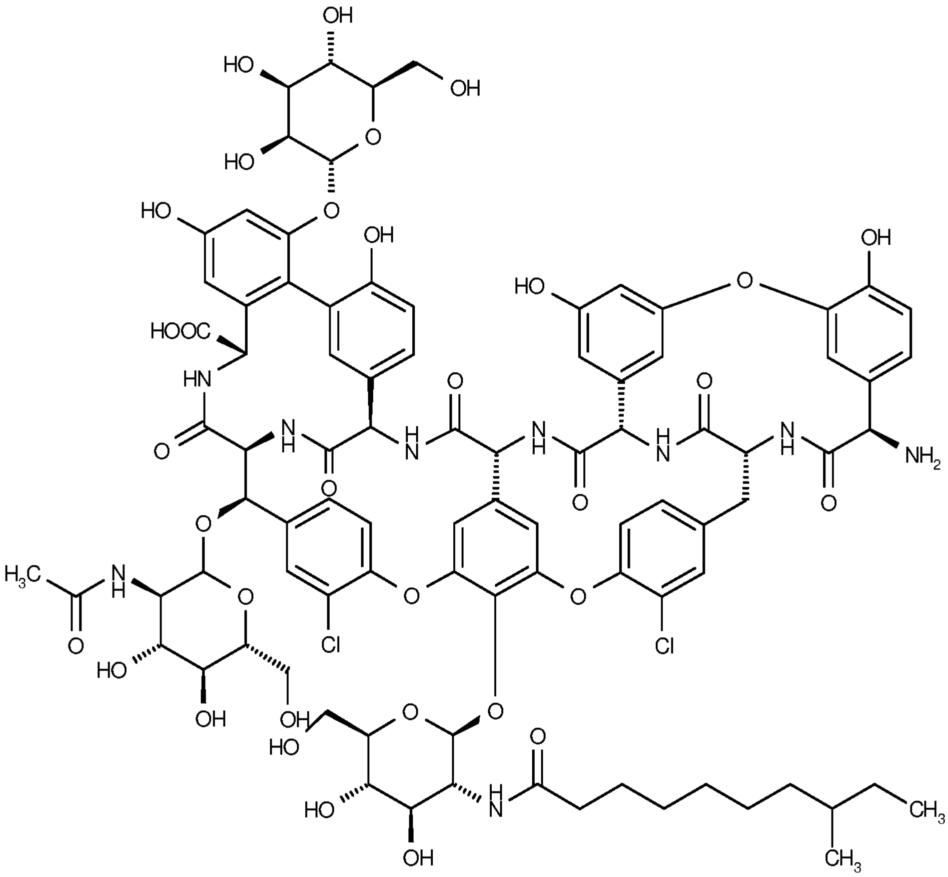

Teicoplanin A2-5

[Chemical Formula 11]

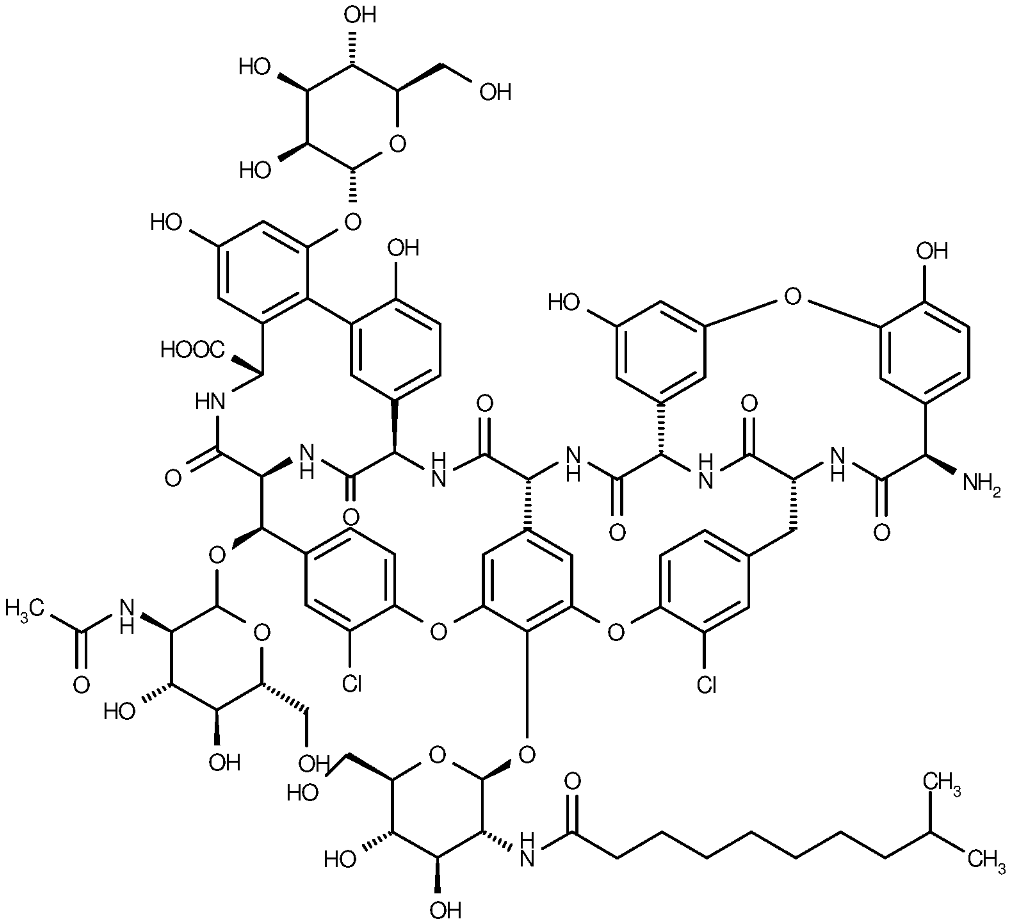

Dalbavancin (BI-397, A-40926)

[Chemical Formula 12]

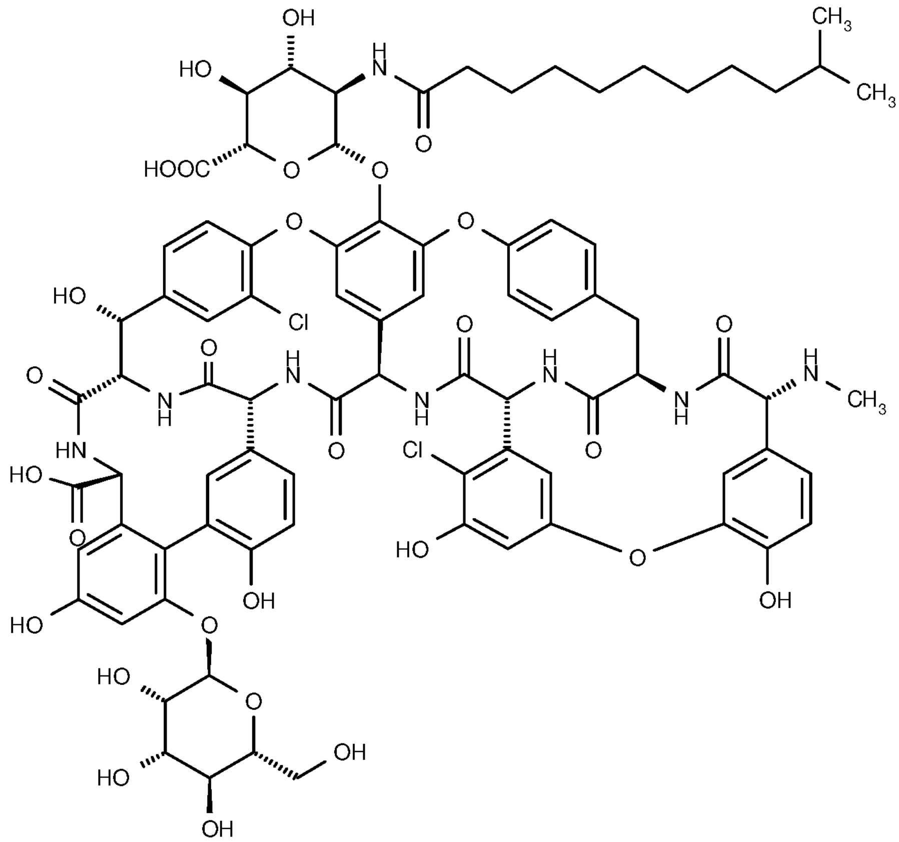

Dalbavancin B0

[Chemical Formula 13]

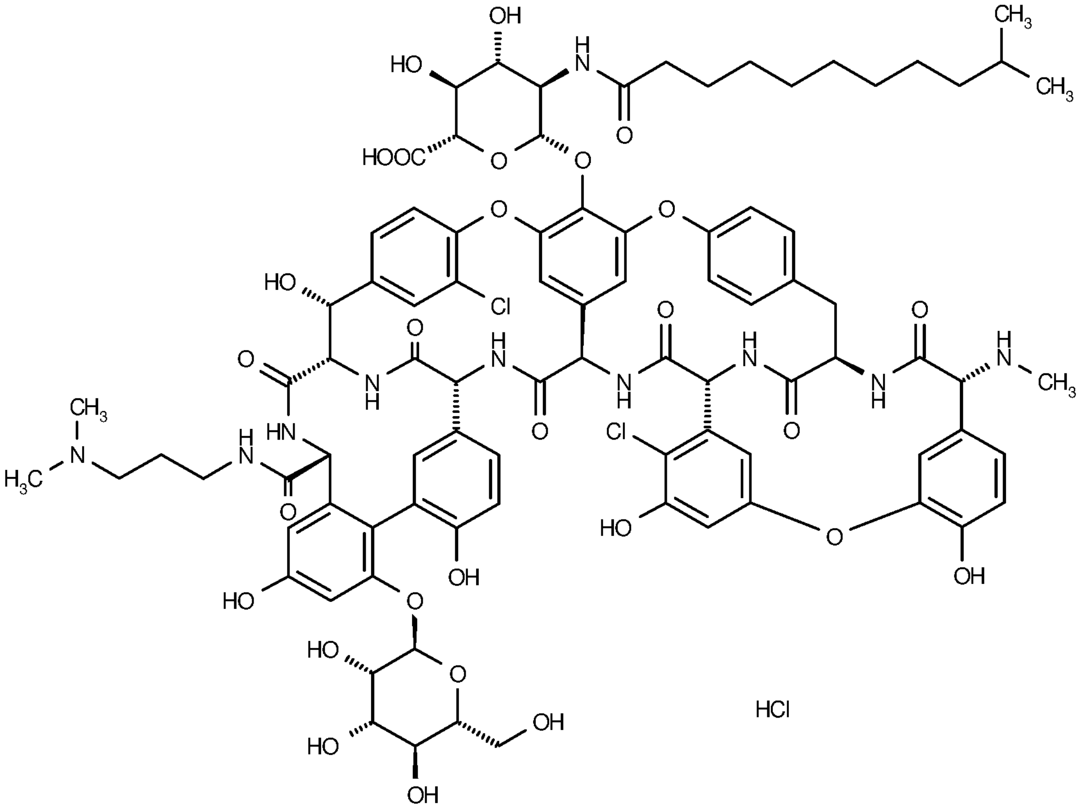

dalbavancin B0

Dalbavancin A0

[Chemical Formula 14]

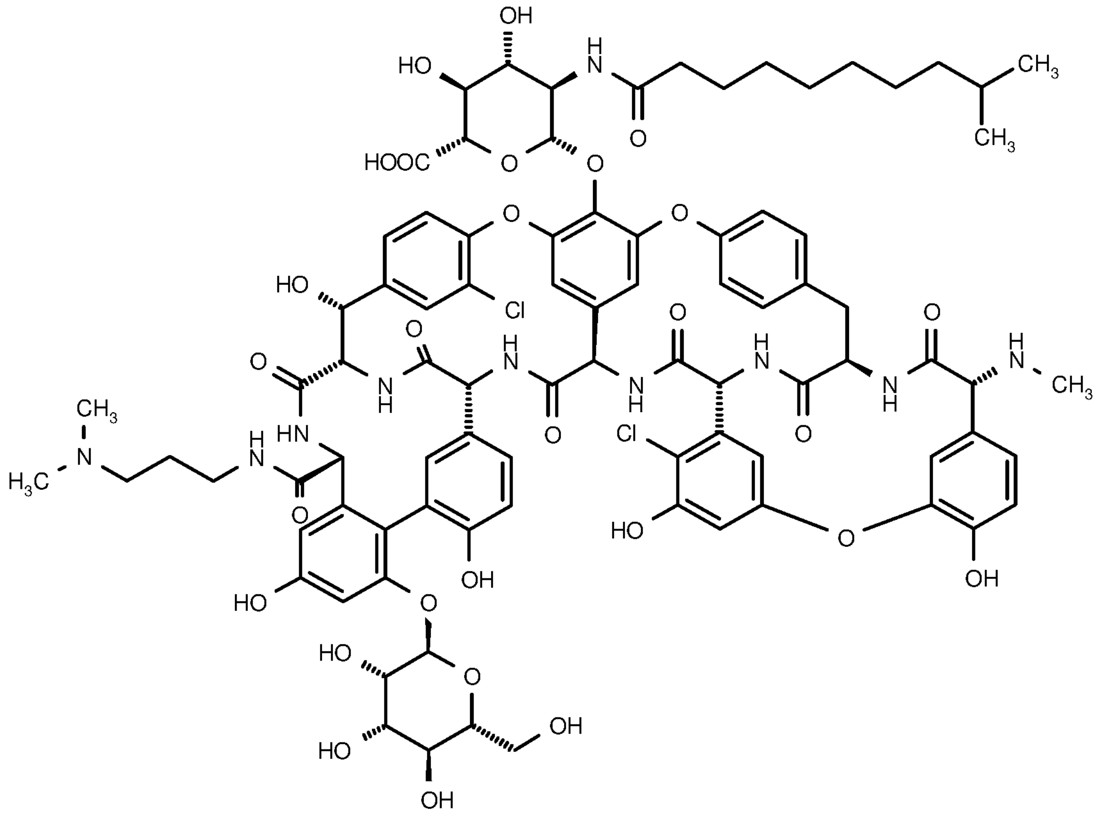

dalbavancin A0

Dalbavancin A1

[Chemical Formula 15]

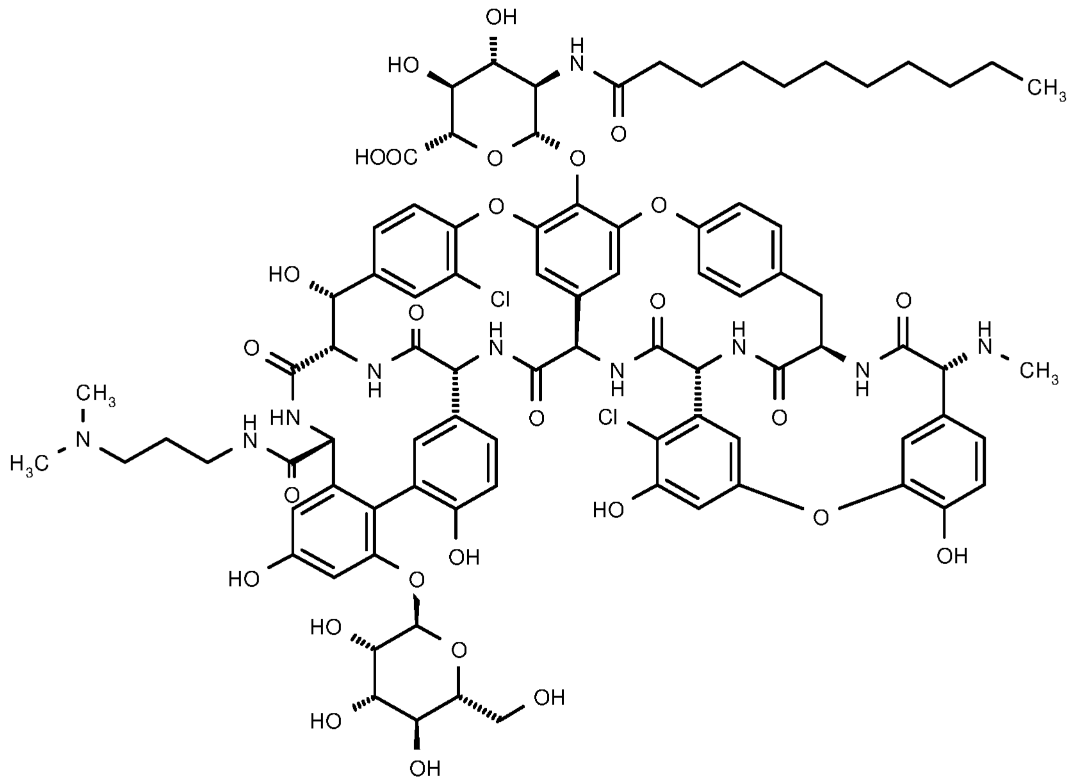

dalbavancin A1

Dalbavancin B1

[Chemical Formula 16]

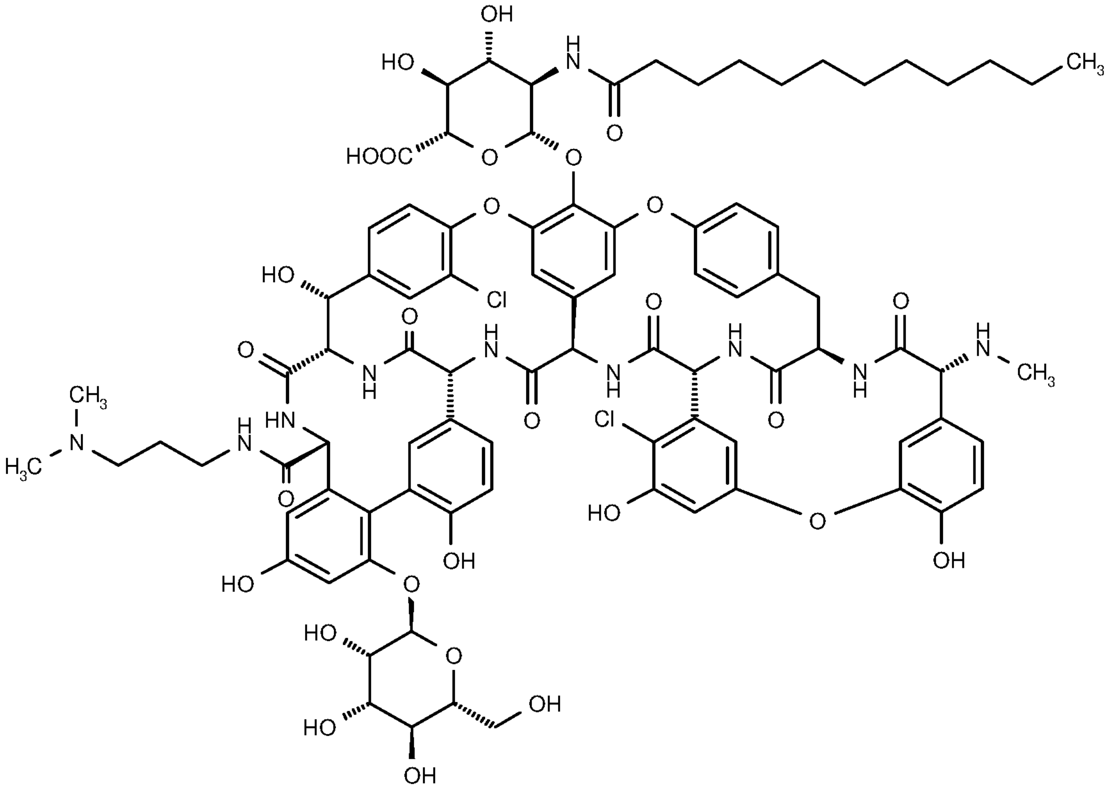

dalbavancin B1

Dalbavancin C0

[Chemical Formula 17]

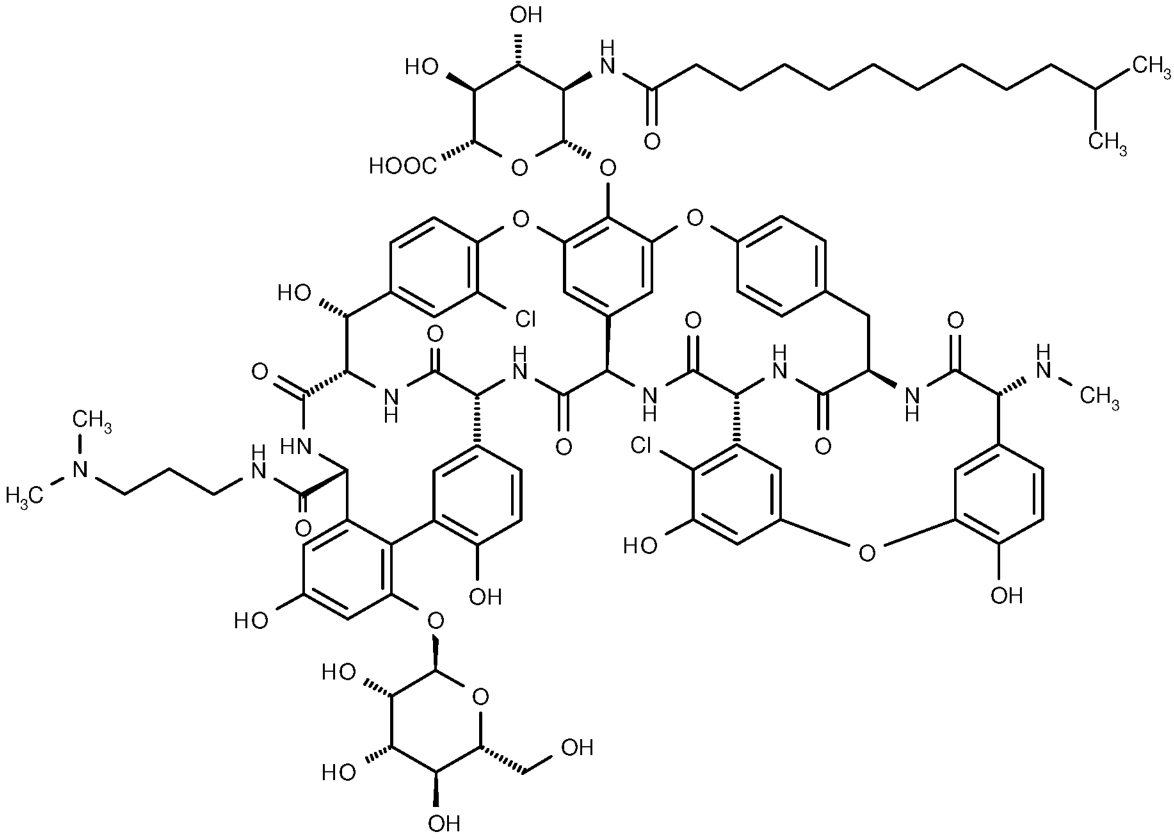

dalbavancin C0

Dalbavancin C1

[Chemical Formula 18]

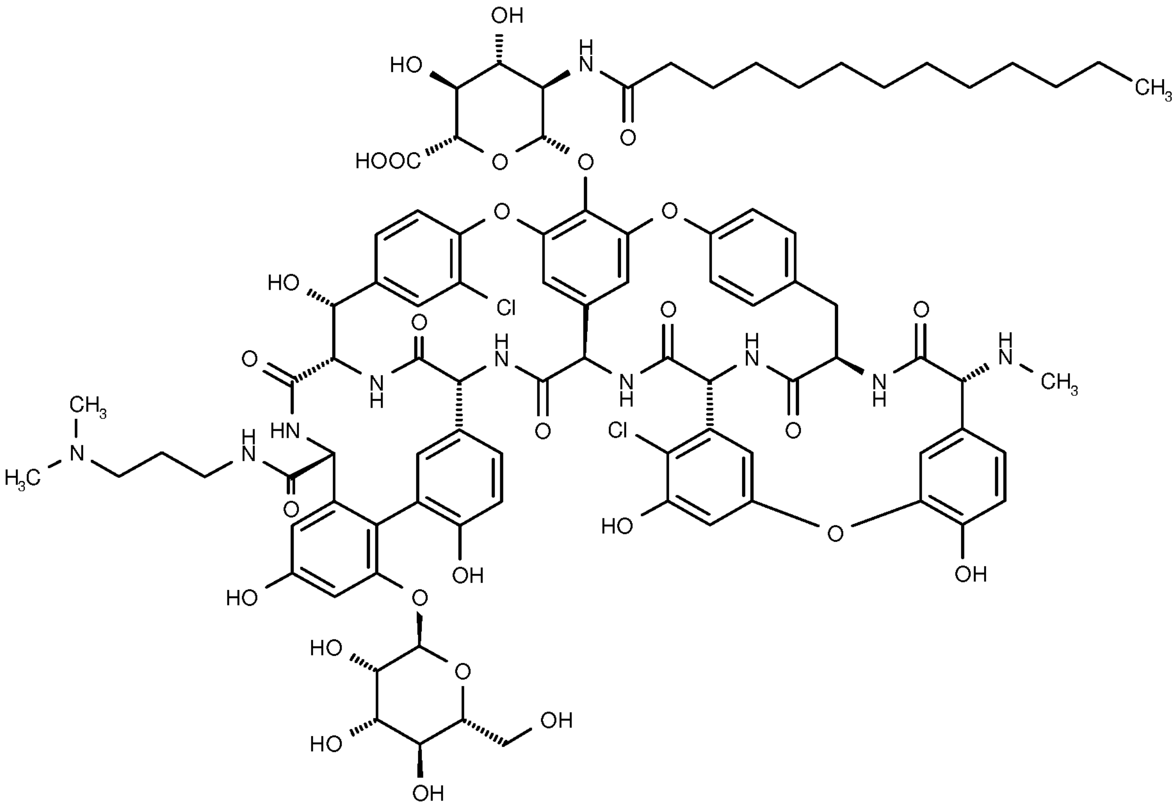

dalbavancin C1

GINA-220

[Chemical Formula 19]

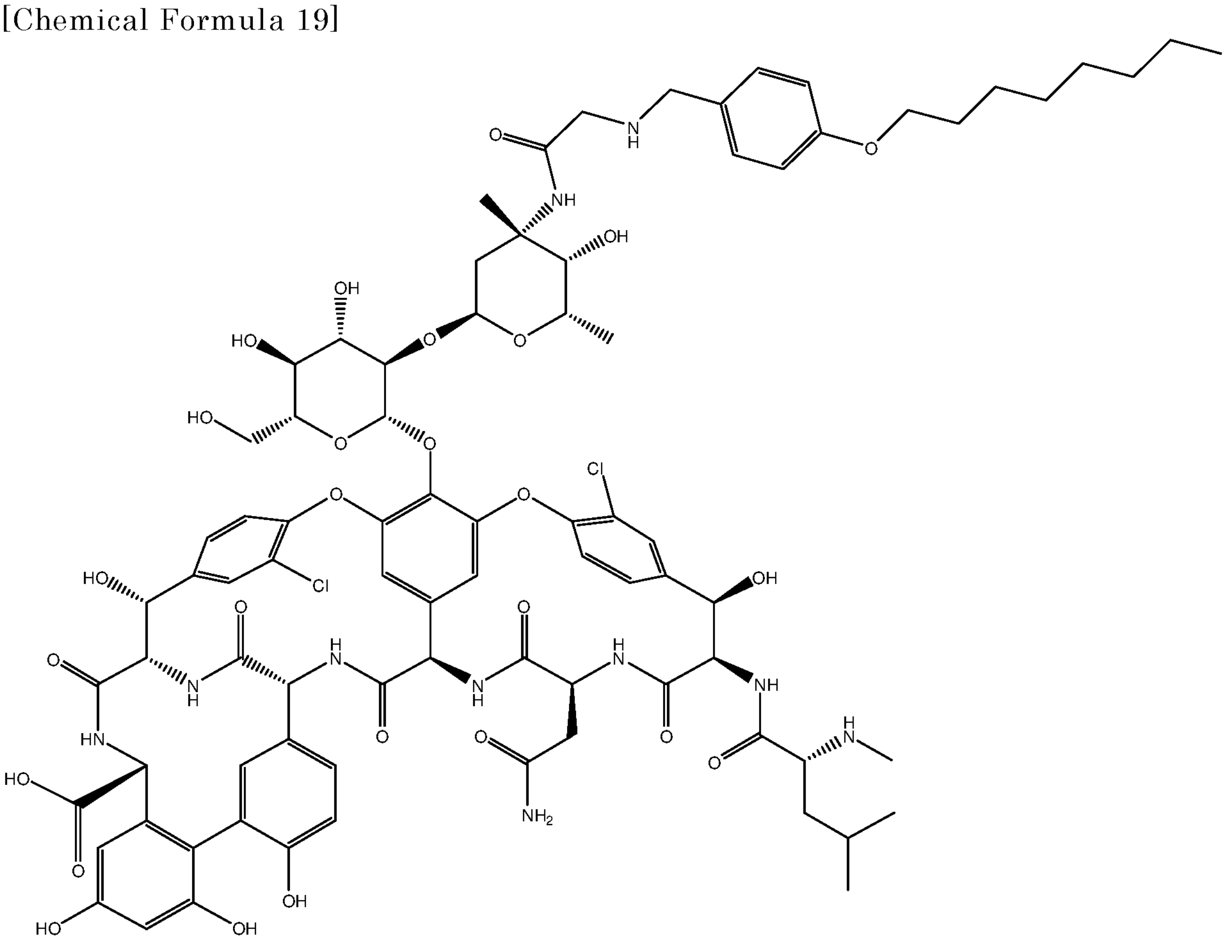

LY191145/LY307599

[Chemical Formula 20]

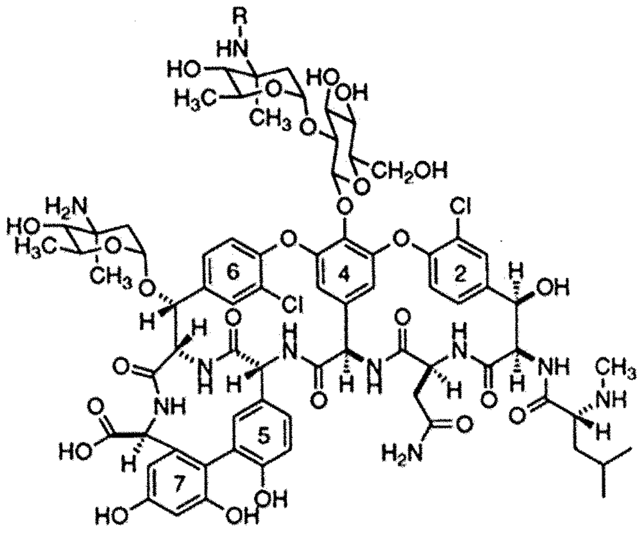

LY191145: R = 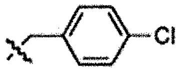

LY307599: R = 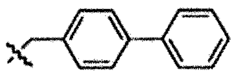

Mideplanin (MDL62873)

[Chemical Formula 21]

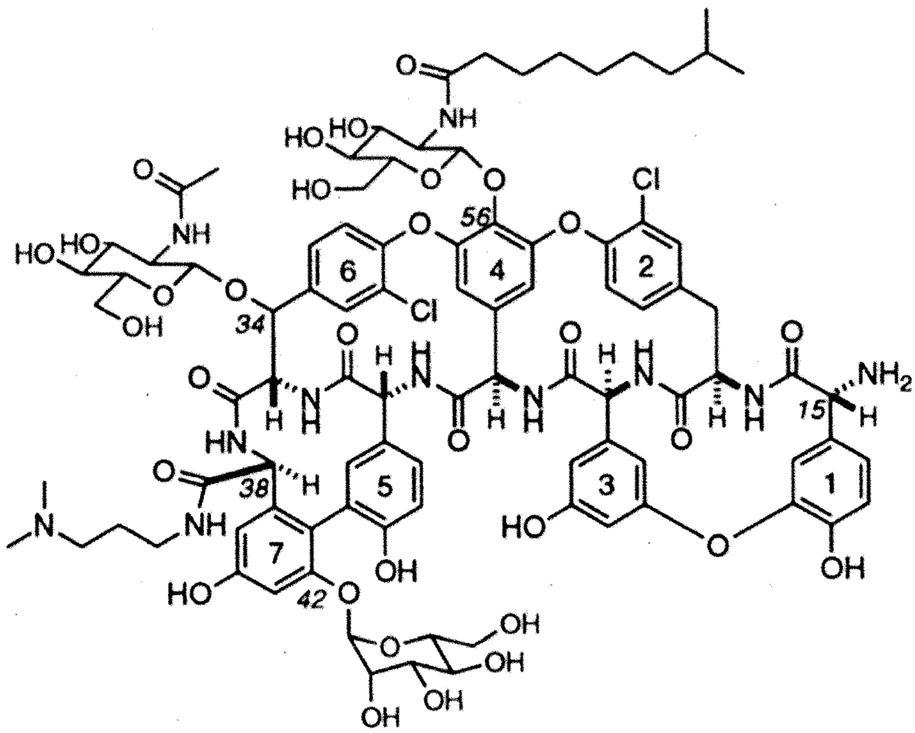

MDL63246/MDL63042

[Chemical Formula 22]

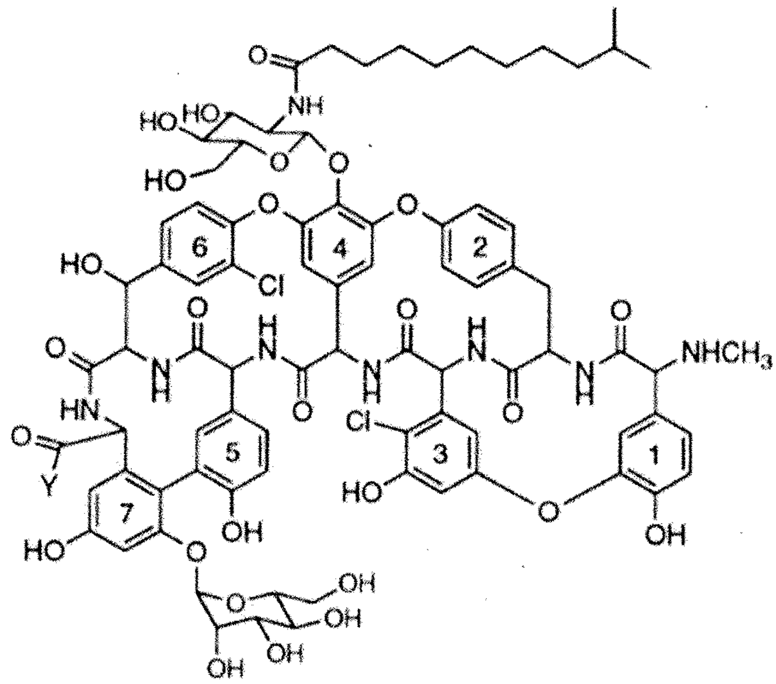

MDL 63,246 Y = 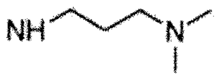

MDL 63,042 Y = 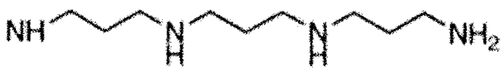

YV11455

[Chemical Formula 23]

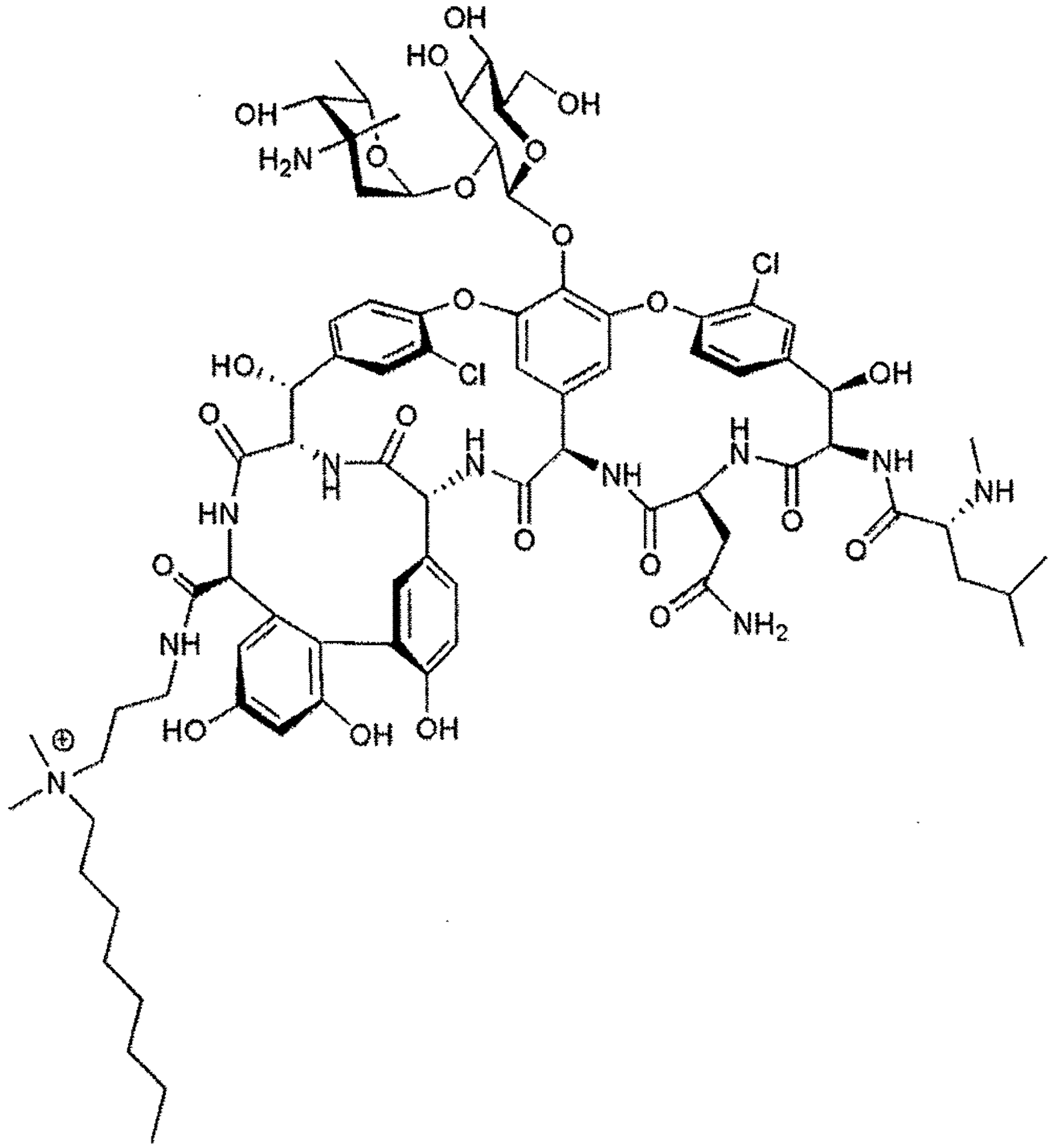

As the antibacterial agent used in the present invention, a cyclic lipopeptide antibacterial agent is used in addition to the glycopeptide antibacterial agent. Examples of the cyclic lipopeptide antibacterial agent include daptomycin, ramoplanin, plusbacin A3, stalobacin I, lysocin E, and derivatives thereof. As the antifungal agent used in the present invention, micafungin sodium, caspofungin acetate, anidulafungin, aminocandin (NXL-201), rezafungin acetate (CD-101), and derivatives thereof are used.

Daptomycin

[Chemical Formula 24]

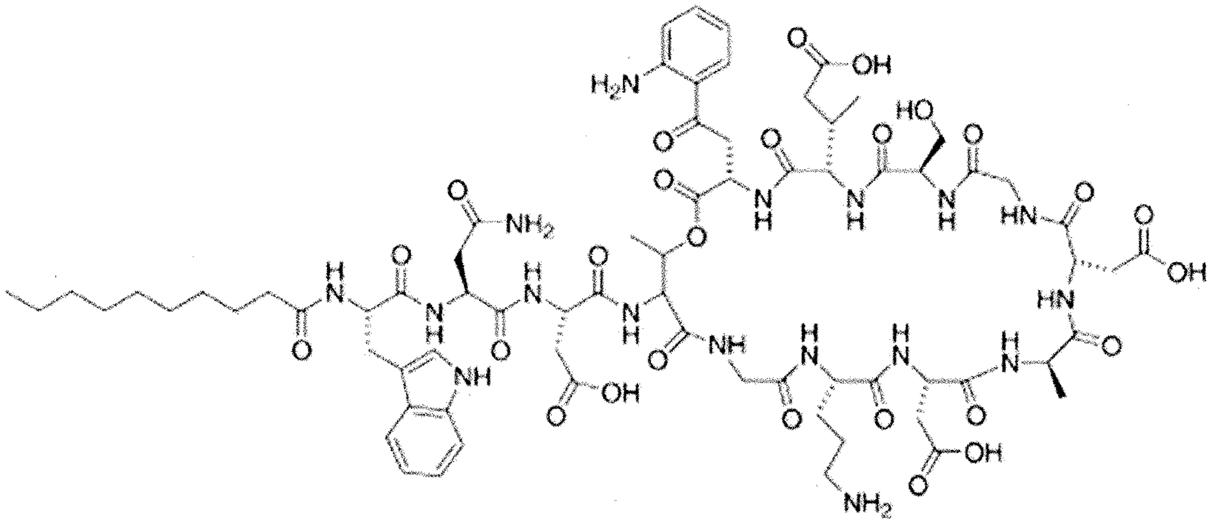

Ramoplanin

[Chemical Formula 25]

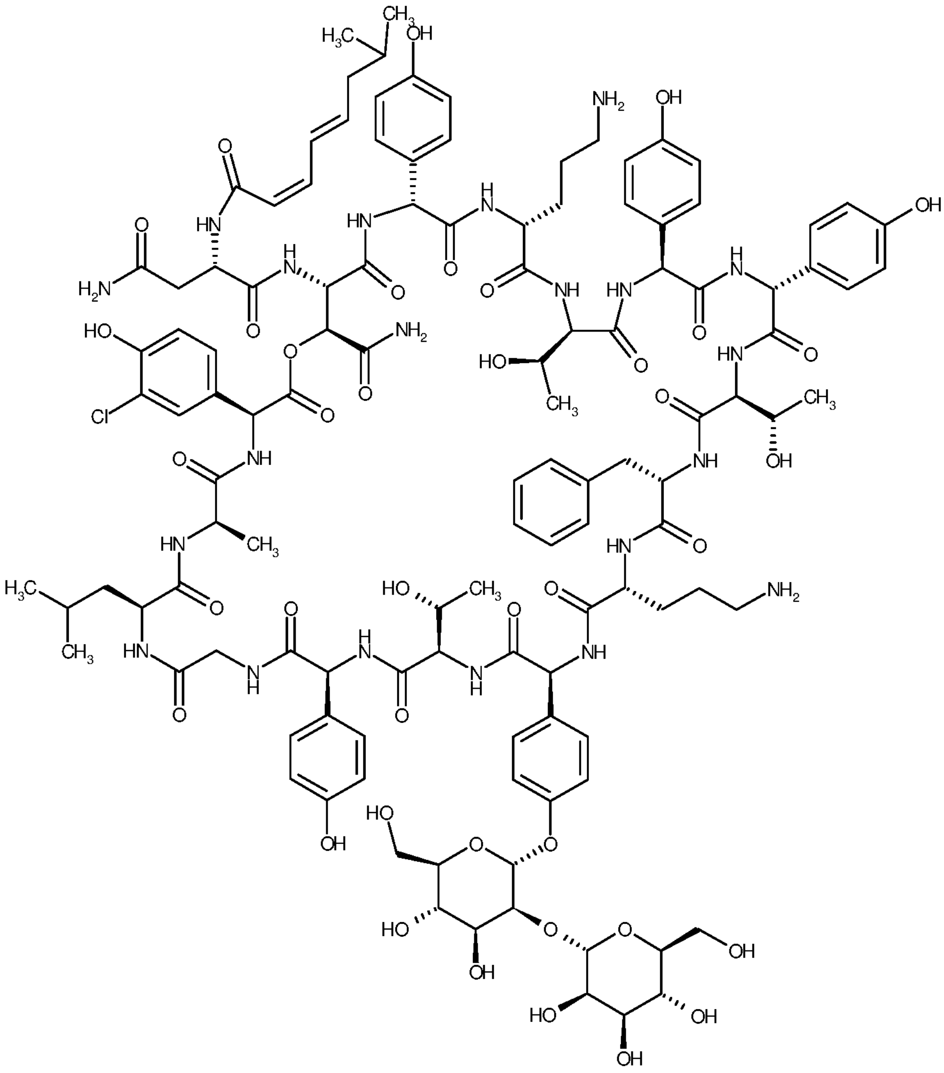

ramoplanin A2

Plusbacin A3

[Chemical Formula 26]

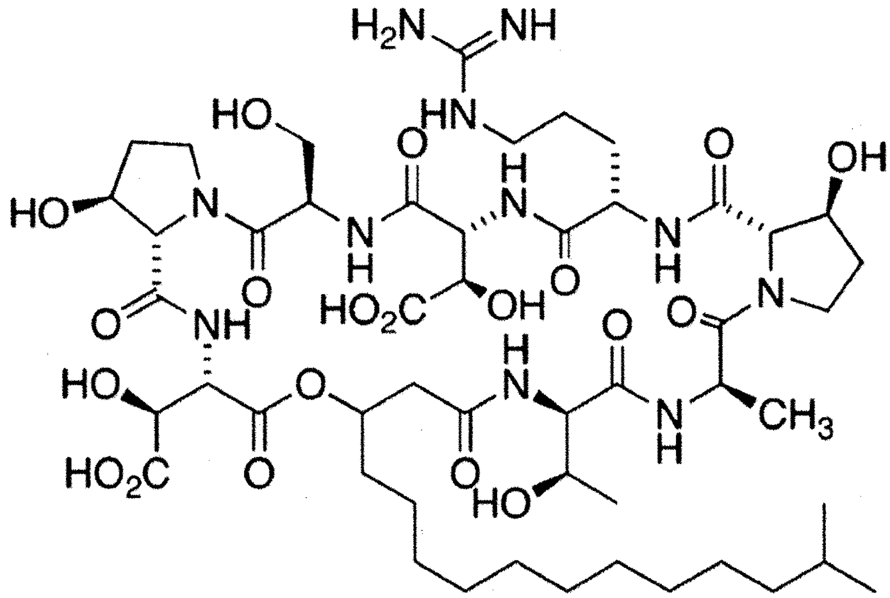

Stalobacin I

[Chemical Formula 27]

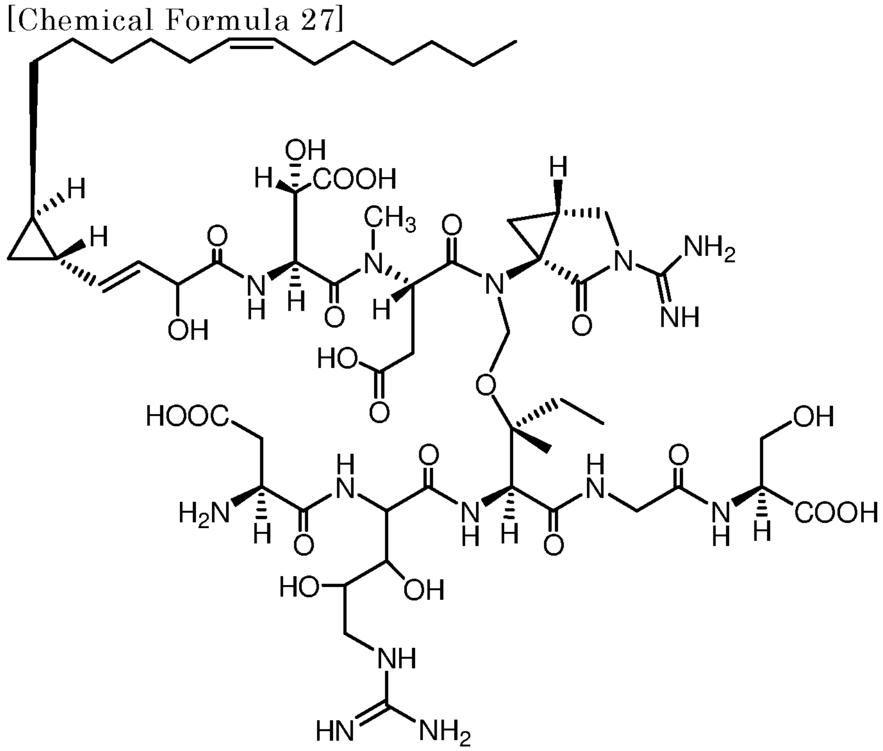

Lysocin E

[Chemical Formula 28]

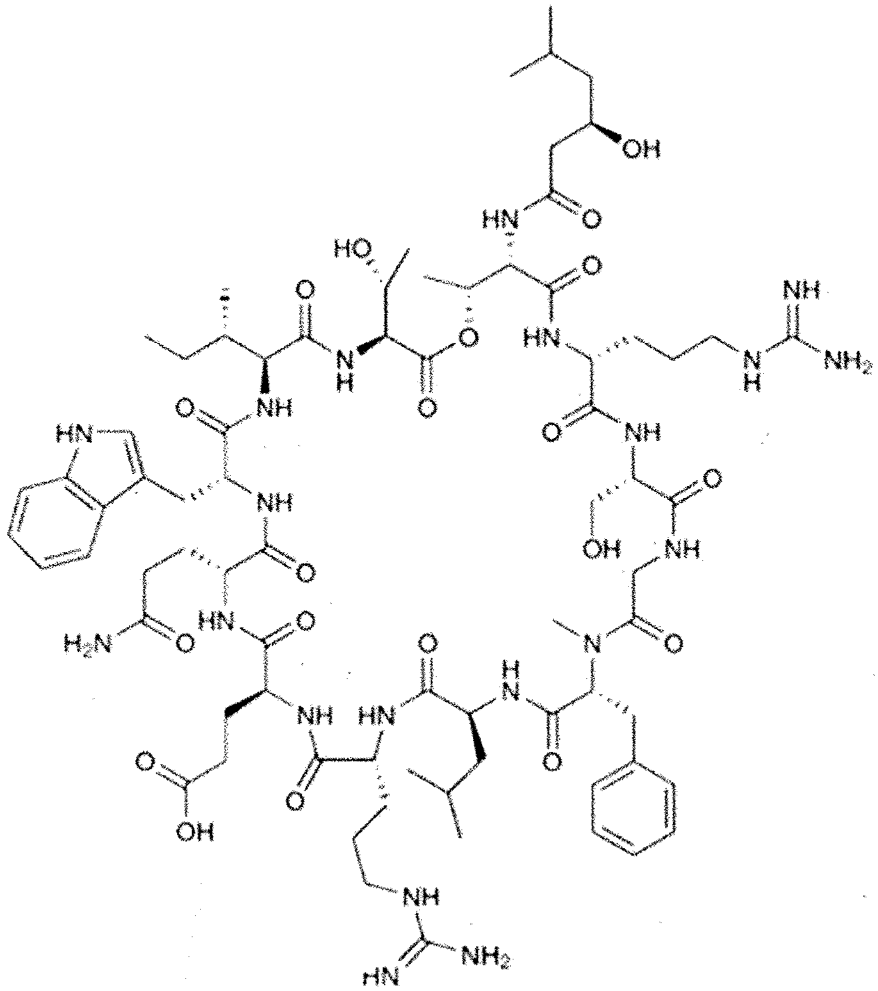

Micafungin sodium

[Chemical Formula 29]

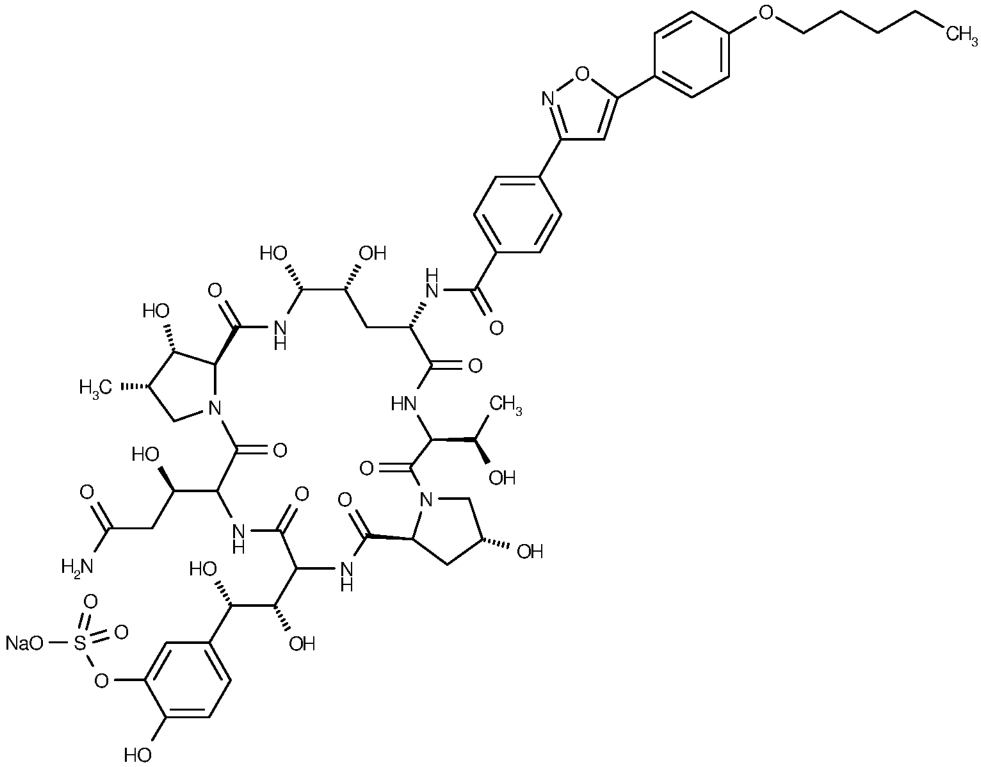

Caspofungin acetate

[Chemical Formula 30]

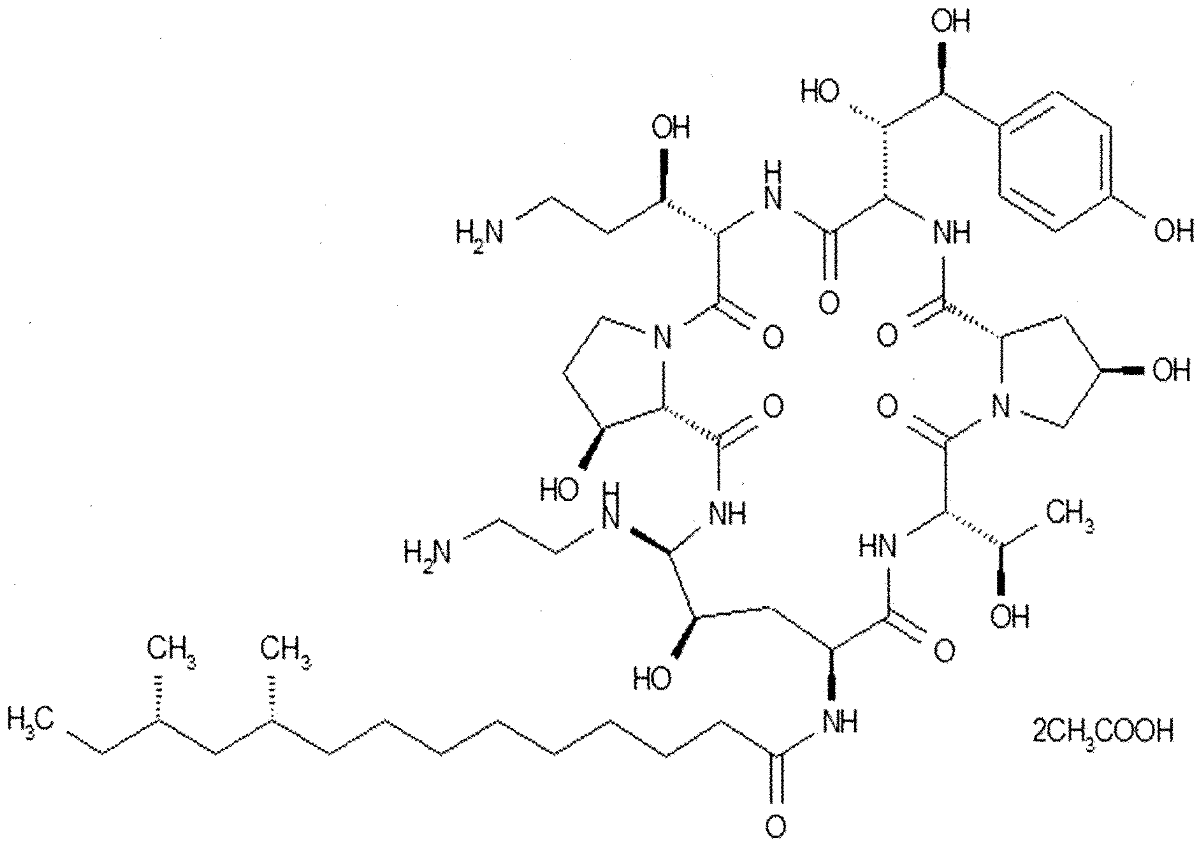

Anidulafungin

[Chemical Formula 31]

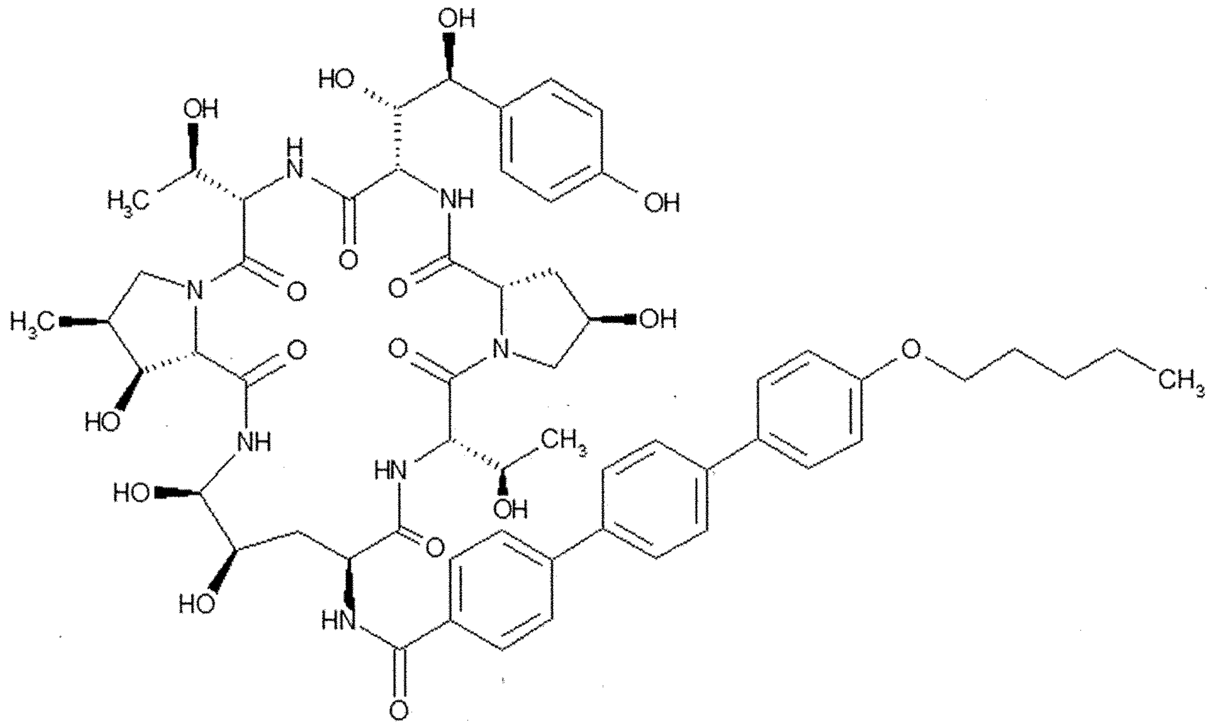

Aminocandin

[Chemical Formula 32]

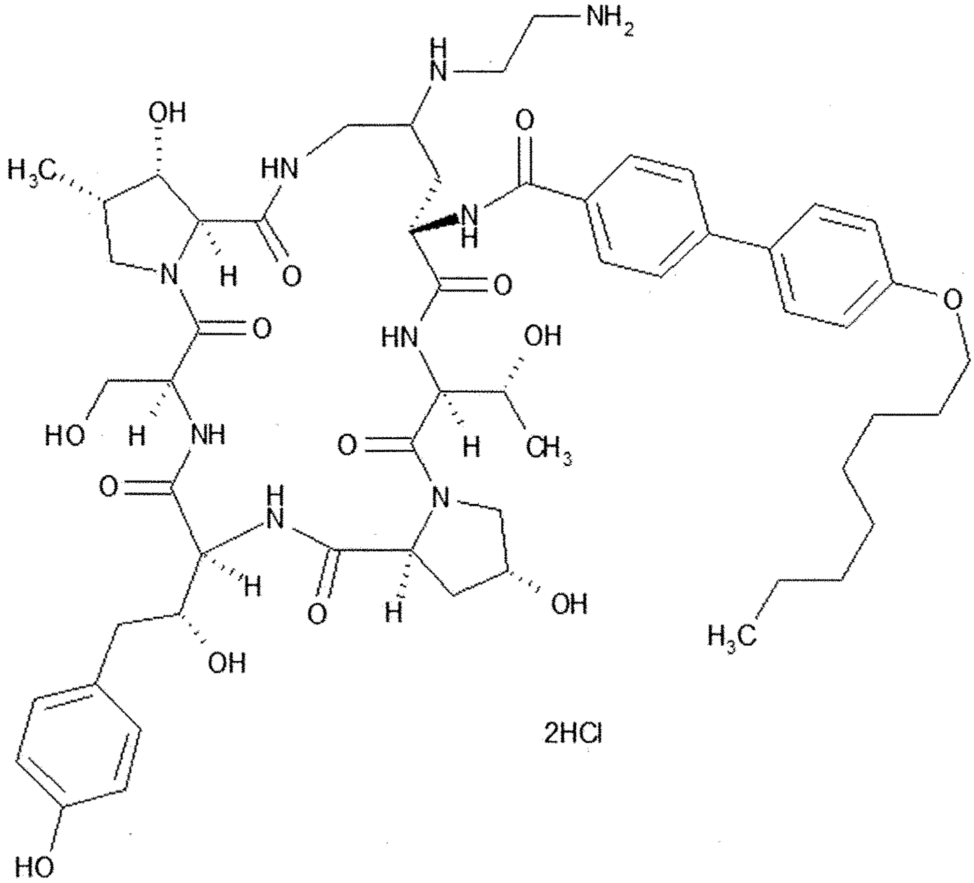

Rezafungin acetate

[Chemical Formula 33]

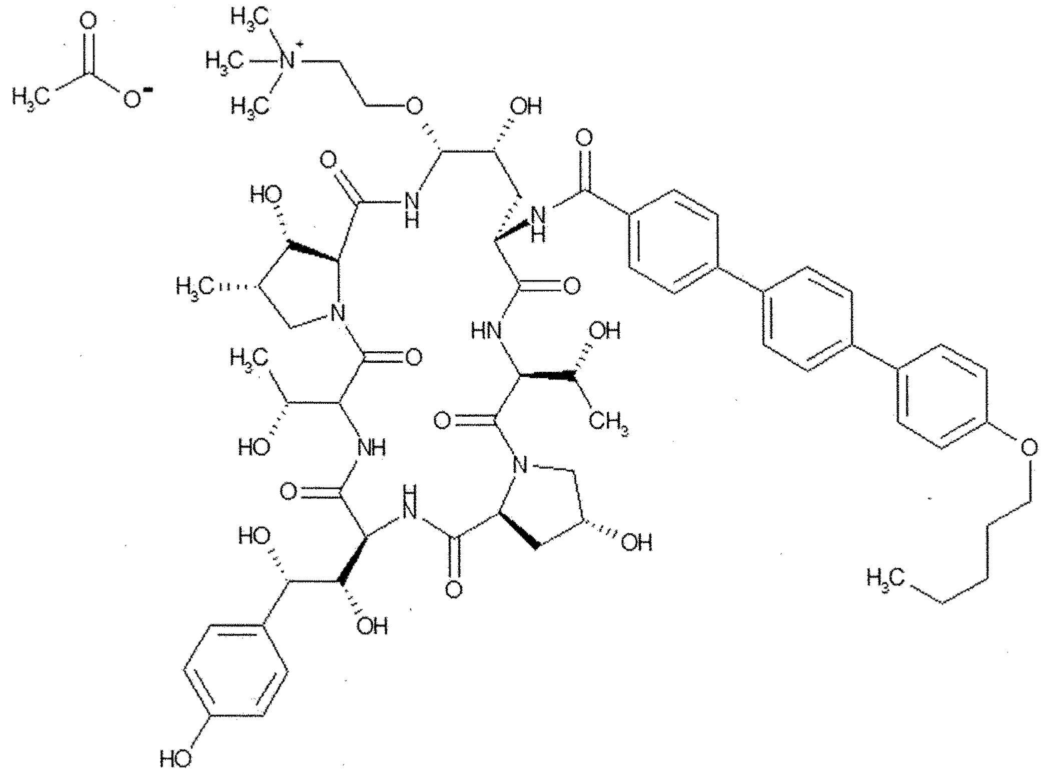

In the present invention, the antibacterial agent is bound to the liposome by a hydrophobic interaction between a lipid-soluble side chain of the antibacterial agent and a phospholipid of the liposome, as shown in Fig. 1. The lipid-soluble side chain of the antibacterial agent is a chemical side chain exhibiting lipid solubility. The lipid-soluble side chain of the antibacterial agent is bound to the phospholipid of the liposome by a lipid-soluble interaction.

Examples of the lipid-soluble side chain include the following side chains.

[Chemical Formula 34]

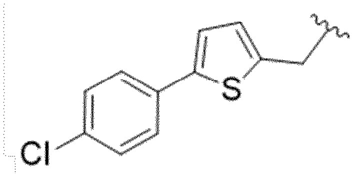

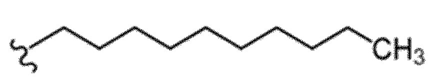

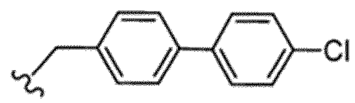

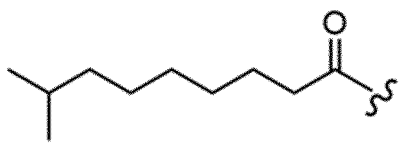

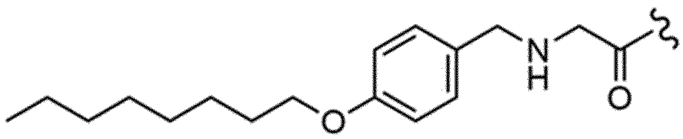

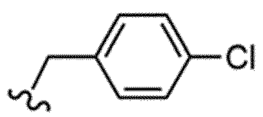

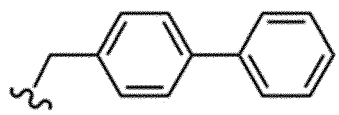

[Chemical Formula 35]

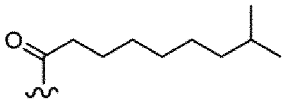
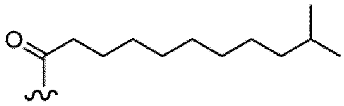
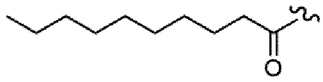
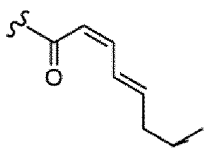
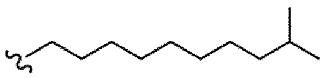
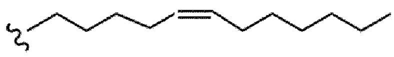
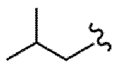
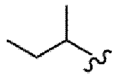
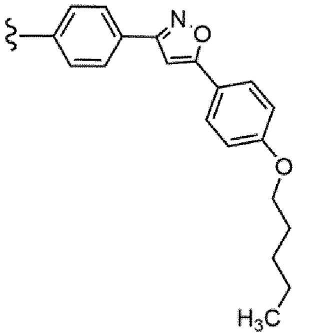
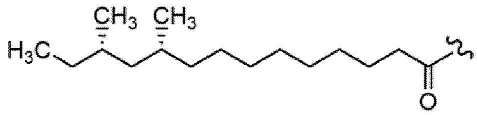
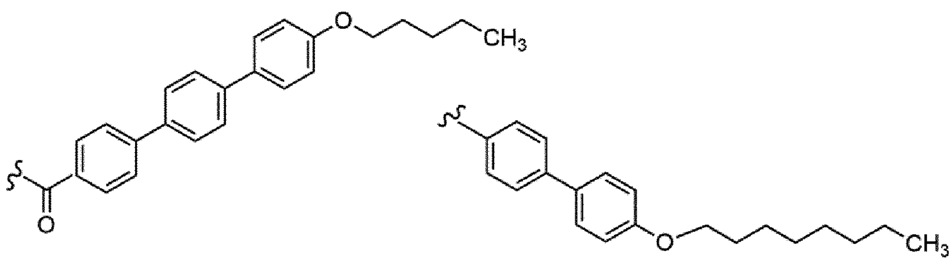
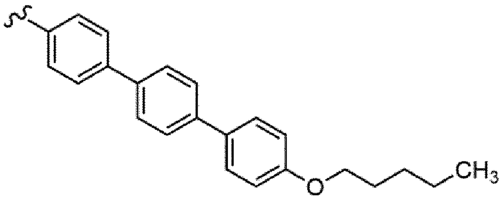
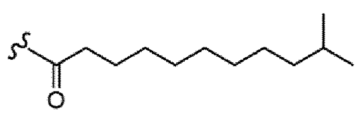
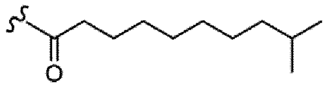
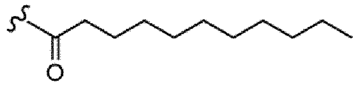

[Chemical Formula 36]

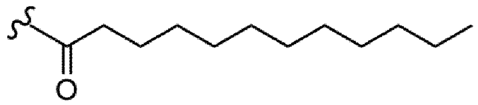
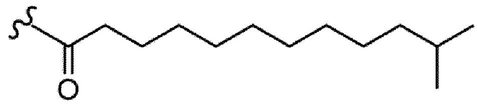
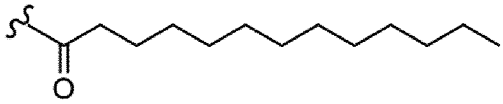
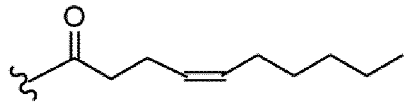
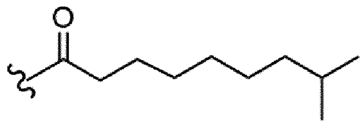
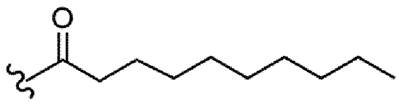
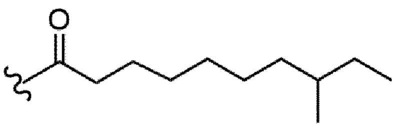
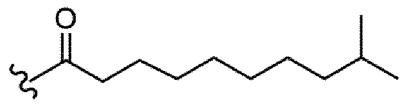
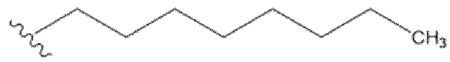

The liposome formulation of the present invention is a liposome formulation in which an antibacterial agent is encapsulated in a liposome and the antibacterial agent is bound outward to a surface layer of the liposome. The antibacterial agent bound outward to the surface layer of the liposome specifically binds to a target molecule of a target pathogenic microorganism, such as methicillin-sensitive Staphylococcus aureus (MSSA) and methicillin-resistant Staphylococcus aureus (MRSA), with 1. a cell wall precursor, 2. a cell membrane, 3. a fungal cell wall synthase, or the like, so that high antibacterial activity can be expressed and toxicity can be reduced.

The liposome formulation of the present invention is a liposome formulation in which an antibacterial agent is encapsulated in a liposome and the antibacterial agent is bound outward to a surface layer of the liposome. However, the liposome formulation can be a positively charged liposome formulation in which a surface charge is positively charged. The liposome formulation of the present invention that is positively charged can express high antibacterial activity and reduce toxicity because after approaching teichoic acid components on surfaces of methicillin-sensitive Staphylococcus aureus (MSSA), methicillin-resistant Staphylococcus aureus (MRSA), and vancomycin-resistant enterococci (VRE) by an electrostatic interaction, the antibacterial agent bound outward to the surface layer of the liposome specifically binds to cell wall precursors of methicillin-sensitive Staphylococcus aureus (MSSA) and methicillin-resistant Staphylococcus aureus (MRSA).

A raw material of the liposome used in a liposome formulation for injection of the present invention may be one that is generally used in a liposome. Specifically, lipids such as a phospholipid, an ether glycerophospholipid, a sphingophospholipid, a glyceroglycolipid, a glycosphingolipid, a glyceride lipid, cardiolipin, a galactolipid, a mannolipid, galactolecithin, and cholesterol; and sterols, tocopherols, and the like as components for stabilizing a liposome membrane are used. In addition, as a raw material of the liposome, an additive such as polyethylene glycol, a saccharide, or a solvent may be used.

More specific examples of the lipid constituting the liposome include egg yolk lecithin, soybean lecithin, hydrogenated egg yolk lecithin, hydrogenated soybean lecithin, a hydrogenated soybean phospholipid (HSPC), soybean-derived phosphatidylcholine, soybean-derived hydrogenated phosphatidylcholine, a purified hydrogenated soybean phospholipid, phosphatidylcholine, egg yolk phosphatidylcholine, hydrogenated soybean phosphatidylcholine, dimyrisylphosphatidylcholine, dipalmitoylphosphatidylcholine, palmitoylstearoylphosphatidylcholine, dioleoylphosphatidylcholine, distearoylphosphatidylcholine, 1,2-dioleoyl-sn-glycero-3-phosphatidylcholine, distearylphosphatidylcholine, 1-palmitoyl-2-oleoyl-sn-glycero-3-phosphatidylcholine, phosphatidylethanolamine, soybean phosphatidylethanolamine, dimyristoylphosphatidylethanolamine, dipalmitoylphosphatidylethanolamine, dioleoylphosphatidylethanolamine, egg yolk phosphatidylethanolamine, monooleoylphosphatidylethanolamine, polyethylene glycol-distearoylphosphatidylethanolamine, phosphatidylglycerol, soybean phosphatidyl glycerol, dimyristoylphosphatidylglycerol, dipalmitoylphosphatidyl glycerol, dioleoylphosphatidyl glycerol, distearylphosphatidylglycerol, dielaidoylphosphatidylglycerol, palmitoylstearoylphosphatidylglycerol, egg yolk phosphatidylglycerol, phosphatidylserine, egg yolk phosphatidylserine, soybean phosphatidylserine, dimyristoylphosphatidylserine, dipalmitoylphosphatidylserine, dioleoylphosphatidylserine, dielaidoylphosphatidylserine, 1,2-dioleoyl-sn-glycero-3-phosphatidylserine, distearoylphosphatidylserine, phosphatidic acid, egg yolk phosphatidic acid, soybean phosphatidic acid, dimyristoylphosphatidic acid, dipalmitoylphosphatidic acid, dioleoylphosphatidic acid, distearylphosphatidic acid, dielaidoylphosphatidylamine, soybean-derived hydrogenated phosphatidylinositol, egg yolk phosphatidylinositol, soybean phosphatidylinositol, dipalmitoylphosphatidylinositol, 1,2-dioleoyl-sn-glycero-3-phosphatidylinositol, dimyristoylphosphatidylinositol, distearoylphosphatidylinositol, cholesterol, cardiolipin, sphingomyelin, hexadecylphosphocholine, N-[1-(2,3-dioleyloxy)propyl]-N,N,N-trimethylammonium chloride, 1,2-bis(oleoyloxy)-3-(trimethylammonio)propane, 1,2-bis(hexadecyloxy)-3-trimethylaminopropane, 3[beta][N-(N'N'-dimethylaminoethane)-carbamyl] cholesterol, 1,2-dimyristroyl-sn-glycero-3-phosphocholine, 1,2-dilauroyl-sn-glycero-3-phosphocholine, 1,2-distearoyl-sn-glycero-3-phosphocholine, 1,2-dimyristoyl-sn-glycero-3-phosphoethanolamine, 1,2-dipalmitoyl-sn-glycero-3-phosphoethanolamine, 1,2-dioleoyl-sn-glycero-3-phosphate monosodium salt, 1,2-dipalmitoyl-sn-glycero-3-[phosphor-rac-(1-glycerol)] sodium salt, 1,2-dimyristoyl-sn-glycero-3-[phospho-L-serine] sodium salt, 1,2-dioleoyl-sn-glycero-3-phosphoethanolamine-N-glutaryl sodium salt, 1,1',2,2'-tetramyristoyl cardiolipin ammonium salt, a glyceride lipid, cardiolipin, a galactolipid, a mannolipid, and galactolecithin, and one or two or more of these lipids may be used.

As the phospholipid, those generally known as phospholipids such as natural phospholipids and synthetic phospholipids can be used. Examples of the phospholipid include phosphatidylcholine, phosphatidylethanolamine, phosphatidylglycerol, phosphatidylserine, phosphatidic acid, and phosphatidylinositol.

Examples of the phosphatidylcholine in (1) include natural phosphatidylcholine such as egg yolk lecithin; and synthetic phosphatidylcholine containing a saturated or unsaturated carboxylic acid having 7 to 22 carbon atoms as a component. Specific examples thereof include egg yolk lecithin, soybean lecithin, hydrogenated egg yolk lecithin, hydrogenated soybean lecithin, a hydrogenated soybean phospholipid (HSPC), hydrogenated lecithin, hydrogenated soybean phosphatidylcholine, soybean-derived phosphatidylcholine (soybean phosphatidylcholine (SPC)), soybean-derived hydrogenated phosphatidylcholine, a purified hydrogenated soybean phospholipid, phosphatidylcholine (PC), egg yolk phosphatidylcholine (EPC), soybean phosphatidylcholine, dimyrisylphosphatidylcholine (DMPC, 1,2-DIMYRISTOYL-SN-GLYCERO-3-PHOSPHOCHOLINE), dipalmitoylphosphatidylcholine (DPPC, 1,2-dipalmitoyl-sn-glycero-3-phosphocholine), palmitoylstearoylphosphatidylcholine (PSPC), dioleoylphosphatidylcholine, distearoylphosphatidylcholine (DSPC), 1,2-dioleoyl-sn-glycero-3-phosphatidylcholine (DOPC), distearylphosphatidylcholine (DSPC, 1,2-DISTEAROYL-SN-GLYCERO-3-PHOSPHOCHOLINE), and 1-palmitoyl-2-oleoyl-sn-glycero-3-phosphatidylcholine (POPC). Examples of the fatty acid residue include groups such as octanoyl, nonanoyl, decanoyl, undecanoyl, lauroyl, myristoyl, palmitoyl, oleyl, stearyl, palmitoleyl, oleyl, linoleyl, linolenyl, and arachidonyl. Fatty acid residue moieties bonded to a 1-position and a 2-position of glycerin may be the same or different.

Examples of the phosphatidylethanolamine (PE) in (2) include natural phosphatidylethanolamine such as soybean-derived phosphatidylethanolamine or soybean-derived hydrogenated phosphatidylethanolamine; and a synthetic type such as phosphatidylethanolamine containing a saturated or unsaturated carboxylic acid having 7 to 22 carbon atoms. Specific examples thereof include phosphatidylethanolamine (PE), soybean phosphatidylethanolamine (SPE), dimyristoylphosphatidylethanolamine, dipalmitoylphosphatidylethanolamine, dioleoylphosphatidylethanolamine (DOPE), egg yolk phosphatidylethanolamine (EPE), monooleoylphosphatidylethanolamine (MOPE), and polyethylene glycol-distearoylphosphatidylethanolamine (PEG-DSPE). Examples of the fatty acid residue include the same groups as in (1) above.

Examples of the phosphatidylglycerol (PG) in (3) include a synthetic type such as phosphatidylglycerol containing a saturated or unsaturated carboxylic acid having 7 to 22 carbon atoms. Specific examples thereof include phosphatidylglycerol (PG), soybean phosphatidylglycerol (SPG), dimyristoylphosphatidylglycerol (DMPG), dipalmitoylphosphatidylglycerol (DPPG), dioleoylphosphatidylglycerol (DOPG), distearylphosphatidylglycerol (DSPG), dielaidoylphosphatidylglycerol (DEPG), palmitoylstearoylphosphatidylglycerol (PSPG), and egg yolk phosphatidylglycerol (Egg-PG). Examples of the constituent fatty acid residue include the same groups as in (1) above.

Examples of the phosphatidylserine (PS) in (4) include a natural type such as soybean-derived phosphatidylserine or soybean-derived hydrogenated phosphatidylserine; and a synthetic type such as phosphatidylserine containing a saturated or unsaturated carboxylic acid having 7 to 22 carbon atoms. Specific examples thereof include phosphatidylserine (PS), egg yolk phosphatidylserine (EPS), soybean phosphatidylserine (SPS), dimyristoylphosphatidylserine (DMPS), dipalmitoylphosphatidylserine (DPPS), dioleoylphosphatidylserine (DOPS), dielaidoylphosphatidylserine (DEPS), 1,2-dioleoyl-sn-glycero-3-phosphatidylserine (DOPS), and distearoylphosphatidylserine (DSPS). Examples of the constituent fatty acid residue include the same groups as in (1) above.

Examples of the phosphatidic acid (PA) in (5) include a synthetic type such as phosphatidic acid containing a saturated or unsaturated carboxylic acid having 7 to 22 carbon atoms. Specific examples thereof include phosphatidic

acid (PA), egg yolk phosphatidic acid (EPA), soybean phosphatidic acid (SPA), dimyristoylphosphatidic acid (DMPA), dipalmitoylphosphatidic acid (DPPA), dioleoylphosphatidic acid (DOPA, 1,2-dioleoyl-sn-glycero-3-phosphatidic acid), distearylphosphatidic acid (DSPA), and dielaidoylphosphatidylamine (DEPA). Examples of the constituent fatty acid residue include the same groups as in (1) above.

Examples of the phosphatidylinositol (PI) in (6) include natural phosphatidylinositol such as soybean-derived hydrogenated phosphatidylinositol, egg yolk phosphatidylinositol (EPI), or soybean phosphatidylinositol (SPI), and dipalmitoylphosphatidylinositol (DPPI), 1,2-dioleoyl-sn-glycero-3-phosphatidylinositol (DOPI), dimyristoylphosphatidylinositol (DMPI), and distearoylphosphatidylinositol (DSPI) can also be used. Examples of the constituent fatty acid residue include the same groups as in (1) above.

Examples of other phospholipids include cardiolipin, sphingomyelin, hexadecylphosphocholine (HEPC), N-[1-(2,3-dioleyloxy)propyl]-N,N,N-trimethylammonium chloride (DOTMA) 1,2-bis(oleoyloxy)-3-(trimethylammonio)propane (DOTAP), 1,2-bis(hexadecyloxy)-3-trimethylaminopropane (BisHOP), 3[beta][N-(N'N'-dimethylaminoethane)-carbamyl] cholesterol (DC-Chol), 1,2-dimyristroyl)-sn-glycero-3-phosphocholine, 1,2-dilauroyl-sn-glycero-3-phosphocholine, 1,2-distearoyl-sn-glycero-3-phosphocholine, 1,2-dimyristoyl-sn-glycero-3-phosphoethanolamine, 1,2-dipalmitoyl-sn-glycero-3-phosphoethanolamine, 1,2-dioleoyl-sn-glycero-3-phosphate monosodium salt, 1,2-dipalmitoyl-sn-glycero-3-[phosphor-rac-(l-glycerol)] sodium salt, 1,2-dimyristoyl-sn-glycero-3-[phospho-L-serine] sodium salt, 1,2-dioleoyl-sn-glycero-3-phosphoethanolamine-N-glutaryl sodium salt, 1,1',2,2'-tetramyristoyl cardiolipin ammonium salt, CHOLESTEROL & LANOLIN PRODUCTS, 1-STEAROYL-SN-GLYCERO-3-PHOSPHOCHOLINE (PTDCHO-MONO180), TRIOCTANOIN, 1,2-DIERUCOYL-SN-GLYCERO-3-PHOSPHOCHOLINE (DEPC), 1-PALMITOYL-2-OLEOYL-SN-GLYCERO-3-PHOSPHOCHOLINE (POPC), DICETYL PHOSPHATE, DODECYLPHOSPHOCHOLINE (DPC), 1-STEAROYL-SN-GLYCERO-3-PHOSPHOCHOLINE (PTDCHO-MONO180), LIPOID PC 14:0/14:0 (1,2-DIMYRISTOYL-SN-GLYCERO-3-PHOSPHOCHOLINE (DMPC)), DICETYL PHOSPHATE, and 1-STEAROYL-SN-GLYCERO-3-PHOSPHOCHOLINE (PTDCHO-MONO180), and one or two or more of these phospholipids may be mixed for use.

Examples of other lipids include a glyceride lipid, cardiolipin, a galactolipid, a mannolipid, and galactolecithin. Examples of the glyceride lipid include a monoglyceride, diglyceride, or triglyceride lipid.

In the present invention, sterols and tocopherols can be used as components for stabilizing a liposome membrane.

It is known that sterol (cholesterol) affects membrane physical properties of a bilayer consisting of a phospholipid. In the case of a bilayer consisting of an unsaturated lipid, when cholesterol is added, fluidity is decreased due to a condensing effect, and in the case of a saturated lipid in a gel phase, the fluidity is increased due to a fluidizing effect.

Examples of the sterols may be those generally known as sterols, and examples thereof include cholesterol, oxysterol, plant sterol, ergosterol, sitosterol, a cationic lipid, cerebroside, sphingosine, ceramide, diacylglycerol, monoacylglycerol, oliacylglycerol, ganglioside, an ether lipid, an alkyl phospholipid, plasmalogen, prostaglandin, campesterol, stigmasterol, and brassicasterol. From the viewpoint of availability and the like, cholesterol is particularly preferable.

The tocopherols may be those generally known as tocopherols, and for example, commercially available $\alpha$-tocopherol is preferable from the viewpoint of availability and the like. Furthermore, a known liposome membrane component capable of constituting a liposome membrane may be contained as long as the effect of the present invention is not impaired.

As the lipid in the liposome, two or more types of lipids may be used. Examples of the combination of two or more types of lipids include a combination of DOPC/DOPG, a combination of DOPC/DPPC, a combination of DOPC/DPPG, a combination of DOPC/cholesterol, a combination of DOPG/DPPC, a combination of DOPG/DPPG, a combination of DOPG/cholesterol, a combination of DPPC/DPPG, a combination of DPPC/cholesterol, a combination of DPPG/cholesterol, a combination of DOPC/DOPG/DPPC, a combination of DOPC/DOPG/DPPG, a combination of DOPC/DOPG/cholesterol, a combination of DOPC/DPPC/DPPG, a combination of DOPC/DPPC/cholesterol, a combination of DOPC/DPPG/cholesterol, a combination of DOPG/DPPC/DPPG, a combination of DOPG/DPPC/cholesterol, a combination of DOPG/DPPG/cholesterol, a combination of DOPC/DPPC/DPPG/cholesterol, a combination of DOPG/DPPC/DPPG/cholesterol, a combination of DOPC/DOPG/DPPC/DPPG, and a combination of DOPC/DOPG/DPPC/DPPG/cholesterol.

As the combination of two or more types of lipids in the liposome, another embodiment includes, for example, a combination of DSPC/DPPG, a combination of DSPC/cholesterol, a combination of DPPG/cholesterol, and a combination of DSPC/DPPG/cholesterol.

As the combination of two or more types of lipids in the liposome, another embodiment includes, for example, a combination of HSPC/DSPG, a combination of HSPC/DPPG, a combination of HSPC/cholesterol, a combination of DSPG/DPPG, a combination of HSPC/DSPG/cholesterol, and a combination of HSPC/DPPG/cholesterol. Here, HSPC can also be replaced by a refined hydrogenated soybean phospholipid.

The lipid constituting the liposome is preferably any combination of two to five selected from DOPC, DOPG, DPPC, DPPG, DSPC, HSPC, DSPG, and cholesterol. More preferred is a combination of DOPC/DOPG/DPPC/DPPG/cholesterol, a combination of DSPC/DPPG/cholesterol, a combination of HSPC/DPPG/cholesterol, a combination of HSPC/cholesterol, or a combination of HSPC/DSPG/cholesterol. More preferred is a combination of HSPC/cholesterol.

In the present invention, in order to improve physicochemical stability of the liposome, a saccharide or a polyhydric alcohol may be added to an internal aqueous phase or an external aqueous phase of the liposome when the liposome is formed.

Examples of the saccharide to be contained in the internal aqueous phase or the external aqueous phase of the liposome include monosaccharides such as glucose, galactose, mannose, fructose, inositol, ribose, and xylose; disaccharides such as saccharose, lactose, cellobiose, trehalose, and maltose; trisaccharides such as raffinose and melezitose; oligosaccharides such as cyclodextrin; polysaccharides such as dextrin; and sugar alcohols such as xylitol, sorbitol, mannitol, and maltitol.

The polyhydric alcohol is not particularly limited as long as it is generally known, and examples thereof include glycerin-based compounds such as glycerin, diglycerin, triglycerin, tetraglycerin, pentaglycerin, hexaglycerin, heptaglycerin, octaglycerin, nonaglycerin, decaglycerin, and polyglycerin; sugar alcohol-based compounds such as sorbitol and mannitol; ethylene glycol, diethylene glycol, triethylene glycol, tetraethylene glycol, pentaethylene glycol, hexaethylene glycol, heptaethylene glycol, octaethylene glycol, and nonaethylene glyco. Among them, glycerin, diglycerin, triglycerin, sorbitol, mannitol, and polyethylene glycol having a molecular weight of 400 to 10000 are preferable from the viewpoint of availability.

Other additives used in the liposome formulation of the present invention include a basic peptide, an alkali metal salt, preferably histidine, sodium phosphate, cholic acid, and deoxycholic acid.

Other examples of the additive used in the liposome formulation of the present invention include vitamin E (tocopherol) and the like as an antioxidant; glycine, arginine, sodium phosphate, sodium citrate, sodium succinate, sodium hydroxide, sodium acetate, hydrochloric acid, and the like as a pH adjusting agent; glucose, sucrose, maltose, trehalose, D-mannitol, D-sorbitol, sodium chloride, and the like as an isotonizing agent; glycerol, dextran, and the like as an excipient; and polysorbate 20, polysorbate 80, and the like as a cryoprotectant.

A weight ratio of the lipid constituting the liposome to the active ingredient (i.e., an antibacterial agent or antifungal agent) in the liposome is 7 : 1 to 20 : 1. Preferred is 15 : 1 to 20 : 1.

The formulation of the present invention can also be used as an oral formulation in addition to an injection and an intravenous fluid formulation.

A method for producing a liposome formulation as the formulation of the present invention may be a method capable of producing a liposome formulation in which an antibacterial agent is encapsulated in the liposome formulation and the antibacterial agent is attached outward to a surface layer of the liposome formulation, more specifically, an antibacterial agent having a lipid-soluble side chain and a liposome are bound to the liposome via the lipid-soluble side chain.

When the liposome formulation as the formulation of the present invention is produced, water or an organic solvent may be used as a solvent. Specific examples of the solvent include water, distilled water, a buffer containing a basic peptide such as histidine, a buffer containing an alkali metal salt such as sodium phosphate, diethyl ether, isopropyl ether, a lower alkanol (e.g., methanol, ethanol, 1-propanol, 2-propanol, or the like), and an aprotic polar solvent (e.g., dimethyl sulfoxide or the like).

Specific examples of the method for producing a liposome formulation include a thin film method, a reverse phase evaporation method, a cholic acid removal method, a French press method, a microreactor method, a remote loading method, a freeze-thaw method, and an ultrasonic method.

The thin film method (Bangham method) is a method in which an aqueous solution is added to a thin film of a lipid and hydrated, whereby the lipid is self-assembled to form a liposome. When a compound to be encapsulated is lipid-soluble, the compound is mixed together with a lipid in advance, and when the compound is water-soluble, the compound can be encapsulated by dissolving the compound in an aqueous solution to be hydrated. Since the formed liposomes are aggregates of different sizes, it is necessary to adjust the size by an extruder or ultrasonic waves according to the purpose. In addition, in the case of removing an unencapsulated matter not encapsulated in the liposome, it is necessary to perform dialysis or ultrafiltration.

The reverse phase evaporation method is a method for producing a liposome using an emulsion. That is, an aqueous phase is added to an organic solvent (diethyl ether, isopropyl ether, and the like) in which phospholipids are dissolved, and ultrasonic treatment is performed to produce a W/O (water-in-oil) emulsion, and then the organic solvent in the W/O (water-in-oil) emulsion is distilled off under reduced pressure. Thereafter, stirring is performed using a mixer to cause phase inversion of the emulsion to form an O/W (oil-in-water) suspension, thereby obtaining a liposome.

The cholic acid removal method is a method for producing a liposome by preparing a phospholipid and a micelle mixture using solubilizing power of a surfactant and removing only the surfactant by a dialysis method. That is, a phospholipid dispersion solution and cholic acid (or deoxycholic acid) are stirred in an aqueous solution to produce a mixed micelle, the mixed micelle is then placed in a dialysis tube, and cholic acid (or deoxycholic acid) is removed in distilled water to obtain a liposome.

The French press method is a method for producing a liposome by extruding a sample against a narrow gap at high pressure. That is, a multilamellar liposome is prepared by the thin film method, and a suspension of the prepared liposome is placed in a French press and extruded by pressure to obtain a liposome.

A coacervation method is a method in which a lower alkanol (good solvent) is selected as a solvent capable of dissolving a phospholipid, and on the other hand, water or a salt solution (poor solvent) is selected as a solvent incapable of dissolving a phospholipid, and these good solvent and poor solvent are mixed to produce a liposome. That is, a phospholipid is dissolved in a lower alkanol (e.g., methanol, ethanol, 1-propanol, 2-propanol, or the like), water or a salt solution as a poor solvent is gradually added to a certain amount of the phospholipid-lower alkanol solution to cause coacervation, and then the lower alkanol contained in the coacervate or the system is dialyzed against a large amount of water to remove the lower alkanol, thereby obtaining a liposome.

The ultrasonic method is a method in which a multilamellar liposome is prepared by the thin film method, and then the multilamellar liposome is irradiated with ultrasonic waves to produce a liposome. In this formulation method, a fine liposome is obtained by preparing a multilamellar liposome or uniformly suspending phospholipids in an aqueous phase, followed by ultrasonic treatment.

As another method for producing a liposome formulation, there is a production method including the following steps. That is, the production method is a method including:

i) mixing a first solution in which one or more lipids are dissolved in a first organic solvent and a second solution in which an antibacterial agent, particularly a glycopeptide antibacterial agent, is dissolved in a second organic solvent to obtain a mixed solution; ii) providing one or more first flows of the solution of the mixed solution obtained in step i), and having a linear velocity of 1.0 m/min or more;

iii) providing one or more second flows of the first aqueous solution, and having a linear velocity greater than or equal to 1.1 m/min; and

iv) combining the one or more first flows and the one or more second flows at a first crossing point to mix components of the first and second flows and providing a first combined flow containing the glycopeptide antibacterial agent-containing lipid nanoparticle.

In the present description, the "first flow" is a first combined flow at a first crossing point outlet in a direction toward an outlet of a production apparatus, and the "second flow" is a flow in a direction different from the first direction.

In the above production method, when the first and second organic solvents are miscible with the first aqueous solution and either the first and second organic solvents or the first aqueous solution contains a buffer, a concentration of the first organic solvent in an outlet solution is less than 60%, preferably less than 50%, and more preferably less than 45%.

The above production method can further include a step of diluting the first combined flow with one or more diluent solvents selected from the group consisting of the first aqueous solution, the second aqueous solution, and water at a second crossing point, and providing a second combined flow.

In the above production method, the first organic solvent may be an organic solvent generally used in production of a liposome, but is preferably a lower alkanol solvent, and more preferably ethanol.

In the above production method, the second organic solvent may be an organic solvent generally used in production of a liposome, but is preferably an aprotic polar solvent, and more preferably dimethyl sulfoxide.

In the above production method, the aqueous solution may be a buffer generally used in production of a liposome, but is preferably a buffer containing a basic peptide and/or an alkali metal salt, more preferably a buffer containing histidine and/or sodium phosphate, and particularly preferably an isotonic buffer obtained by adding a basic peptide and/or an alkali metal salt to an isotonic solution such as 10% sucrose.

Further, the above production method includes a step of incubating from a step of obtaining the first combined flow to before the second crossing point.

Further, the above production method includes a step of cooling to room temperature, dialyzing, and concentrating the incubated outlet solution.

By the above production method, about 80% or more of the antibacterial agent is encapsulated in a lipid nanoparticle in the outlet solution.

As a solvent for further dispersing or suspending the liposome formulation of the present invention, an aqueous solvent such as distilled water, distilled water for injection, physiological saline, or a buffer such as a phosphate buffer, a carbonate buffer, a Tris buffer, or an acetate buffer, or the like can be used.

A liposome suspension obtained by suspending the liposome of the present invention in a liquid can then be pulverized by a treatment such as vacuum drying or freeze drying.

The liposome has multiple lipid molecules. Each of the lipid molecules has a hydrophilic moiety and a hydrophobic moiety. When the liposome is placed in a predetermined dispersion medium (e.g., water), the lipid molecules form a double layer (hereinafter, it may be referred to as a "lipid bilayer"), and a shape of the liposome becomes spherical

such that a surface area is minimized. In the lipid bilayer, the hydrophilic moiety constitutes an outermost layer (hereinafter, it may be referred to as an "outermost hydrophilic layer") and an innermost layer (hereinafter, it may be referred to as an "innermost hydrophilic layer"). The hydrophobic moiety constitutes two layers sandwiched between the innermost hydrophilic layer and the outermost hydrophilic layer (hereinafter, a layer on an innermost hydrophilic layer side may be referred to as a "first hydrophobic layer", and a layer on an outermost hydrophilic layer side may be referred to as a "second hydrophobic layer"). A form and structure of the liposome formulation of the present invention can be performed by confirming an aggregation state and a lamellar structure of the liposome formulation. As an image analysis method, transmission electron microscopy, cryo-electron microscopy, atomic force microscopy, or the like can be used.

A particle size distribution of the liposome formulation of the present invention can be represented by a quantitative index such as a figure and a polydispersity index together with a mean (average particle size) or a median. The liposome is a generally spherical hollow particle surrounded by a lipid bilayer. The average particle size of the liposome is, for example, 10 to 500 nm, preferably 20 to 300 nm, and more preferably 30 to 120 nm. As another preferred embodiment, the average particle size is 60 to 120 nm. Preferred is 60 to 80 nm. More preferred is 65 to 75 nm. The liposome may be a liposome having a particle size of less than 100 nm (SUV: small unilameller vesicle), a liposome having a particle size of 100 nm or more (LUV: large unilameller Vesicle), or a multilamellar liposome (MLV: multilameller vesicle). As a method for testing the particle size distribution, a dynamic light scattering meter is mainly used, but a laser diffraction method, a nanoparticle tracking analysis method, or an electric resistance nanopulse method may be used. When a dynamic light scattering meter is used, it is necessary to select an appropriate distribution display (such as a number-based distribution or a volume-based distribution).

A surface charge (zeta potential) of the liposome formulation of the present invention is an important property because it affects in vivo clearance, tissue distribution, and uptake into cells. Since the surface charge cannot be directly measured due to an influence of an electric double layer formed on a surface of the liposome by a counter ion in an aqueous solution, the surface charge is generally evaluated and examined as a zeta potential. As a test method, electrophoretic light scattering (laser Doppler method) is mainly used. The zeta potential of the liposome formulation of the present invention may be a zeta potential of a normal liposome formulation, but preferably a positively charged liposome formulation.

Thermodynamic properties of a membrane of the liposome formulation of the present invention can be evaluated by differential scanning calorimetry, temperature dependence in fluorescence spectrum properties of a lipid membrane-inserted fluorescent probe, and the like. Even in a liposome in which a clear phase transition temperature is not determined by a content of cholesterol, an active ingredient having high lipid solubility, or the like, thermodynamic properties such as exothermic and endothermic profiles are useful as indices representing fluidity and uniformity of a lipid bimolecular membrane.

A titer of the liposome formulation of the present invention can be measured by a calorimeter, a microcalorimeter, a quartz crystal microbalance method, and a surface plasmon resonance analysis method.

With respect to in vitro release properties of the active ingredient from the liposome formulation of the present invention, releasability of the active ingredient from the liposome should be demonstrated using physiologically and/or clinically relevant solvents and with stirring as necessary.

An osmotic pressure after formulation of the liposome formulation of the present invention is desirably isotonic (about 280 mOsm/kg) in order to prevent rupture and shrinkage of a liposome structure.

With respect to an encapsulation rate of the active ingredient in the liposome formulation of the present invention, a method can be used in which an active ingredient encapsulated in the liposome and a free active ingredient are separated by solid phase extraction, size exclusion chromatography, an ultracentrifugation method, a gel filtration method, a dialysis method, or the like, and then an amount of the active ingredient in each fraction is quantified by high performance liquid chromatography or a spectrophotometer. In addition, it is possible to quantify lipids (phosphatidylcholine and the like) in the formulation, correct a drug recovery rate based on the calculated lipid recovery rate, and calculate the encapsulation rate of the active ingredient in the liposome formulation.

[EXAMPLES]

The present invention will be described in more detail below by way of Examples, Comparative Examples, and Reference Examples, but the present invention is not limited thereto. Formulations of Examples, Comparative Examples, and Reference Examples were produced by the following methods.

(1) Examination of lipid

1) Formulation

Lipid components of each formulation, mixing (weight) ratios of the lipid components, and mixing (weight) ratios

of a total amount of the lipid components and a drug are shown in Tables 1 to 4. As drugs, compound A, telavancin (manufactured by Shionogi & Co., Ltd.), and oritavancin (manufactured by Shanghai Sun-shine Chemical Technology Corporation Limited) were used. As lipids in a liposome, 1,2-dioleoyl-sn-glycero-3-phosphocholine (DOPC, manufactured by Nippon Fine Chemical Co., Ltd.), dioleoylphosphatidylglycerol (sodium 1,2-dioleoyl-sn-glycero-3-phospho-rac-(1-glycerin) (DOPG, manufactured by Nippon Fine Chemical Co., Ltd.), dipalmitoylphosphatidylcholine (1,2-dipalmitoyl-sn-glycero-3-phosphocholine) (DPPC, manufactured by Nippon Fine Chemical Co., Ltd.), dipalmitoylphosphatidylglycerol (DPPG, manufactured by Nippon Fine Chemical Co., Ltd.), cholesterol (manufactured by Nippon Fine Chemical Co., Ltd.), distearylphosphatidylcholine (DSPC, manufactured by Nippon Fine Chemical Co., Ltd.), a hydrogenated soybean phospholipid (HSPC, manufactured by Nippon Fine Chemical Co., Ltd.), and distearylphosphatidylglycerol (DSPG, manufactured by Nippon Fine Chemical Co., Ltd.) were used.

[Table 1]

| | Lipid component (drug: compound A) | Mixing ratio (molar ratio or weight ratio) of lipid component (total amount) |
|---|---|---|
| Example 2 | DOPC/DOPG/DPPC/DPPG/ cholesterol | 1/1/1/1/1 (molar ratio) |
| Example 3 | DSPC/DPPG/cholesterol | 3/1.5/1 (weight ratio) |

Table 2]

| | Lipid component (drug: telavancin) | Mixing ratio (weight ratio) of lipid component (total amount) |
|---|---|---|
| Example 4 | HSPC/DPPG/cholesterol | 3/1.5/1 (weight ratio) |
| Example 5 | HSPC/cholesterol | 3/1 (weight ratio) |

[Table 3]

| | Lipid component (drug: oritavancin) | Mixing ratio (weight ratio) of lipid component (total amount) |
|---|---|---|
| Example 1 | HSPC/cholesterol | 3/1 (weight ratio) |
| Example 6 | HSPC/DSPG/Cholesterol | 3/1.5/1 (weight ratio) |
| Example 7 | HSPC/DPPG/cholesterol | 3/1.5/1 (weight ratio) |
| Example 8 | HSPC/DPPG/cholesterol Flow rate: 1/4 of Example 7 | 3/1.5/1 (weight ratio) |

[Table 4]

| | Mixing ratio of total amount of lipid component and drug (total amount of lipid component/drug, weight ratio) |
|---|---|
| Example 1 | 20/1 |

2) Method for producing formulation

Hereinafter, a method for producing a liposome formulation of Example 1 and Example 3 will be described.

(Example 1)

HSPC and cholesterol were dissolved in ethanol (Nacalai Tesque, Inc.) at a lipid concentration of 33.5 mg/ml to prepare a lipid solution. Oritavancin was dissolved in dimethyl sulfoxide (Nacalai Tesque, Inc.) at a drug concentration of 62.5 mg/ml, and then mixed with the lipid solution in an ultrasonic warm bath at 85°C to prepare an oritavancin/lipid mixed solution. Using a stainless thin tube having an inner diameter of 1.0 mm, the oritavancin/lipid mixed solution was

fed at a linear velocity of 10.2 m/min, and a histidine buffered 10% sucrose solution (pH 6.5) was fed at a linear velocity of 46.4 m/min by a liquid feeding pump. After preheating by feeding 10 m (oritavancin/lipid solution) and 20 m (L-histidine buffered 10% sucrose solution) under warming at 85°C, the two solutions were mixed with a T-shaped mixer at a collision angle of 90°. After mixing, incubation was performed by feeding 10 m under warming at 85°C to prepare a liposome stock solution having an ethanol concentration of 18%. The prepared liposome stock solution was allowed to stand at room temperature until the liquid temperature decreased to room temperature.

Ethanol was removed from the liposome stock solution and the stock solution was concentrated by tangential flow filtration using KrosFlo (registered trademark) KR2i TFF System (manufactured by Repligen Corporation) and a hollow fiber membrane module (115 $cm^2$, 500 kD, manufactured by Repligen Corporation) to prepare a liposome solution. Using a histidine buffered 10% sucrose solution (pH 6.5) as a buffer for liquid exchange, liquid exchange was performed until an amount of a permeate liquid was 10 times an amount of the liposome solution.

(Example 3)

DSPC, DPPG, and cholesterol were dissolved in ethanol (Nacalai Tesque, Inc.) at a lipid concentration of 130.9 mg/ml to prepare a lipid solution. Compound A was dissolved in dimethyl sulfoxide (Nacalai Tesque, Inc.) at a drug concentration of 45 mg/ml, and then mixed with the lipid solution in an ultrasonic warm bath at 85°C to prepare a compound A/lipid mixed solution.

Using a stainless thin tube having an inner diameter of 1.0 mm, the compound A/lipid mixed solution was fed at a linear velocity of 10.19 m/min, and a histidine buffered 10% sucrose solution (pH 6.5) was fed at a linear velocity of 17.8 m/min by a liquid feeding pump. After preheating by feeding 10 m (compound A/lipid solution) and 20 m (L-histidine buffered 10% sucrose solution) under warming at 85°C, the two solutions were mixed with a first T-shaped mixer at a collision angle of 90°. The mixed solution was further fed by 10 m under warming at 85°C, and then mixed with a histidine buffered 10% sucrose solution (pH 6.5) at a linear velocity of 28.0 m/min at a collision angle of 90° with a second T-shaped mixer at room temperature to prepare a liposome stock solution having an ethanol concentration of 18%. The prepared liposome stock solution was allowed to stand at room temperature until the liquid temperature decreased to room temperature.

Ethanol was removed from the liposome stock solution and the stock solution was concentrated by tangential flow filtration using KrosFlo (registered trademark) KR2i TFF System (manufactured by Repligen Corporation) and a hollow fiber membrane module (115 $cm^2$, 500 kD, manufactured by Repligen Corporation) to prepare a liposome solution. Using a histidine buffered 10% sucrose solution (pH 6.5) as a buffer for liquid exchange, liquid exchange was performed until an amount of a permeate liquid was 10 times an amount of the liposome solution. In Examples 7 and 8, the drug, a type of the lipids, and a mixing ratio of the lipids were the same, but a flow rate in Example 8 was set to 1/4 of a flow rate in Example 7.

3) Measurement of drug content (drug encapsulation rate) in liposome formulation

A drug content in the produced formulation was measured with a high performance liquid chromatograph mass spectrometer (LCMS-8050, Shimadzu Corporation). Conditions of liquid chromatography and the mass spectrometer are as follows.

(Conditions of liquid chromatography)
Column: Cosmosil Packed Column 5C18-AR- II (4.6 mm I.D. × 150 mm)
Column oven temperature: 25°C
Mobile phase A: 0.1% formic acid aqueous solution
Mobile phase B: 0.1% formic acid-containing acetonitrile solution
Flow rate: 1.0 mL/min
Gradient: 0 min: B12%, 10 min: B20%, 15 min: B90%, 20 min: B12%
Injection amount: 10 pL
Autosampler temperature: 4°C
Measurement time: 20 minutes

[Conditions of mass spectrometer]

Ionization mode: ESI (positive mode)
Nebulizer gas: 3.0 L/min

Drying gas: 10 L/min
Desolvation line temperature: 250°C
Block heater: 400°C
Measurement mode: single ion monitoring (SIM)
Quantitative ion: compound A (m/z = 838), telavancin (m/z = 823), oritavancin (m/z = 897)

A measurement sample was prepared by adding Triton X-100 so as to have a final concentration of 0.1%, and diluting the mixture 100 to 500 times with a diluent (88% mobile phase A + 12% mobile phase B). As a quantitative method, an absolute calibration curve method was used, and as a standard solution for a calibration curve, one obtained by adjusting each drug substance to 0.1, 0.25, 0.5, 1.0, 1.5, or 2.0 pg/ml with the diluent was used. In addition, as a result of a test for adding and recovering the standard solution to an empty liposome solution (lipid concentration 333 pg/ml: 200 pg/ml HSPC, 66.5 pg/ml MPEG2K-DSPE, 66.5 pg/ml cholesterol), average recovery rates were compound A: 105%, oritavancin: 109%, and telavancin: 102%.

The drug content (drug encapsulation rate) was calculated by quantifying phosphatidylcholine in the formulation by phospholipid-C test Wako (Wako Pure Chemical Industries, Ltd.) and correcting the drug recovery rate based on the calculated lipid recovery rate.

4) Method for measuring particle size

The liposome solution was diluted 200 times with PBS(-) (manufactured by Sigma-Aldrich Co. LLC), and then an average particle size and a particle size distribution were measured by a dynamic light scattering method using Zetasizer nanoZS (manufactured by Malvern Panalytical).

5) Method for measuring membrane potential

The liposome solution was diluted 50 times with water for injection (manufactured by Otsuka Pharmaceutical Factory, Inc.), and then a zeta potential was measured by an electrophoresis method using Zetasizer nanoZS (manufactured by Malvern Panalytical).

(Experimental results)

The average particle sizes and the membrane potentials of Examples 1 to 8 are shown in Tables 5 to 7. As a result, all of the average particle sizes were around 100 nm. The membrane potentials of the formulations of Examples 2 to 4 and 6 to 8 were all negatively charged. On the other hand, the membrane potential of the formulation of Example 5 containing telavancin as a drug and HSPC and cholesterol as lipids was negatively charged, but an absolute value thereof was low. Furthermore, the membrane potential of the formulation of Example 1 containing oritavancin as a drug and HSPC and cholesterol as lipids was positively charged. In the formulations of Examples 7 and 8, the drug content of Example 8 with a reduced flow rate was reduced and the average particle size was increased.

[Table 5]

| | Lipid component and mixing ratio thereof (drug: compound A) | Drug content (%) | Average particle size (nm) | Membrane potential (mV) |
|---|---|---|---|---|
| Example 2 | DOPC/DOPG/DPPC/DPPG/ cholesterol = 1/1/1/1/(molar ratio) | 90.8 | 91.08 | -50.0 |
| Example 3 | DSPC/DPPG/cholesterol = 3/1.5/1 (weight ratio) | 85.5 | 69.8 | -45.6 |

[Table 6]

| | Lipid component and mixing (weight) ratio thereof (drug: telavancin) | Drug content (%) | Average particle size (nm) | Membrane potential (mV) |
|---|---|---|---|---|
| Example 4 | HSPC/DPPG/cholesterol = 3/1.5/1 (weight ratio) | 81.7 | 75.75 | -57.7 |

(continued)

| | Lipid component and mixing (weight) ratio thereof (drug: telavancin) | Drug content (%) | Average particle size (nm) | Membrane potential (mV) |
|---|---|---|---|---|
| Example 5 | HSPC/cholesterol = 3/1 (weight ratio) | 78.7 | 119.1 | -2.64 |

[Table 7]

| | Lipid component and mixing (weight) ratio thereof (drug: oritavancin) | Drug content (%) | Average particle size (nm) | Membrane potential (mV) |
|---|---|---|---|---|
| Example 1 | HSPC/cholesterol = 3/1 (weight ratio) | 100.2 | 70.3 | +17.1 |
| Example 6 | HSPC/DSPG/cholesterol = 3/1.5/1 (weight ratio) | 81.0 | 91.15 | -46.7 |
| Example 7 | HSPC/DPPG/cholesterol = 3/1.5/1 (weight ratio) | 91.7 | 61.5 | -50.4 |
| Example 8 | HSPC/DPPG/cholesterol = 3/1.5/1 (weight ratio), flow rate: 1/4 as ratio to Example 7 | 80.6 | 116.8 | -68.3 |

(2) Plasma concentration of active ingredient after administration of liposome formulation

Plasma concentrations after administration of the liposome formulations of Examples 4 and 5 (drug: telavancin) and the liposome formulations of Examples 1 and 6 to 8 (drug: oritavancin) were examined by the following methods.

(Examined experimental materials and methods)

1) Animals used: ICR mice were used.
2) Breeding conditions: The mice were allowed to freely take chow and sterilized tap water.
3) Dose and grouping setting: A predetermined dose was intravenously administered. The intravenous dose was 5 mg/kg (n = 3).
4) Formulation of dosing solution: The dosing solution was administered as a 5% glucose solution.
5) Administration method: Administration was performed via the tail vein through a syringe with an injection needle.
6) Evaluation item: Blood was collected over time, and the plasma concentration of the compound of the present invention was measured using LC/MS/MS.
7) Parameter analysis: For the plasma concentration of the compound of the present invention, area under the plasma concentration-time curve (AUC), systemic clearance (CLtot), elimination half-life (t1/2), and steady state distribution volume (Vdss) of the compound of the present invention were calculated by non-compartmental model analysis.

.

(Experimental results)

Changes in plasma concentration are shown in Figs. 2 and 3, and plasma parameters are shown in Tables 8 to 10. As a result, in the case of telavancin, as shown in Fig. 2 and Table 8, the formulations of Examples 4 and 5, which are liposome formulations, had higher blood retention and AUC and a longer elimination half-life as compared with administration of the drug substance alone, and particularly, the liposome formulation of Example 5, which had a lower negative charge of a membrane potential, had higher AUC and a longer elimination half-life. In addition, in the case of oritavancin, as shown in Fig. 3 and Tables 9 and 10, the formulations of Examples 1 and 6, which are liposome formulations, had higher blood retention and AUC and a longer elimination half-life as compared with administration of the drug substance alone, and particularly, the liposome formulation of Example 1, which had a positively charged membrane potential, had higher blood retention. The liposome formulations of Examples 7 and 8 containing oritavancin were not

so different in blood retention from the drug substance.

PK parameters after administration of telavancin drug substance and formulations of Examples 4 and 5

[Table 8]

| | Drug substance | Example 4 | Example 5 |
|---|---|---|---|
| AUC inf or all (μg·hr/mL) | 57200 ± 12800 | 443000 ± 170000 | 524000 ± 23700 |
| CL tot (mL/min/kg) | 1.52 ± 0.32 | 0.209 ± 0.083 | 0.159 ± 0.007 |
| T1/2 (hr) | 2.7 ± 2.7 | 7.1 ± 2.5 | 10.5 ± 1.5 |
| Vdss (mL/hr) | 0.134 ± 0.016 | 0.099 ± 0.011 | 0.130 ± 0.013 |

PK parameters after administration of oritavancin drug substance and formulations of Examples 1 and 6

[Table 9]

| | Drug substance | Example 1 | Example 6 |
|---|---|---|---|
| AUC inf or all (μg·hr/mL) | 86900 ± 3350 | 500000 ± 72400 | 787000 ± 13700 |
| CL tot (mL/min/kg) | 0.989 ± 0.049 | 0.169 ± 0.026 | 0.106 ± 0.002 |
| T1/2 (hr) | 2.2 ± 1.3 | 11.3 ± 2.0 | 4.2 ± 0.6 |
| Vdss (mL/hr) | 0.109 ± 0.023 | 0.140 ± 0.016 | 0.030 ± 0.006 |

PK parameters after administration of formulations of Examples 7 and 8

[Table 10]

| | Example 7 | Example 8 |
|---|---|---|
| AUC inf or all (μg·hr/mL) | 52400 ± 5680 | 80100 ± 10800 |
| CL tot (mL/min/kg) | 1.62 ± 0.22 | 1.05 ± 0.13 |
| T1/2 (hr) | 3.5 ± 1.3 | 4.6 ± 0.3 |
| Vdss (mL/hr) | 0.183 ± 0.033 | 0.145 ± 0.001 |

(3) Tissue distribution of active ingredient after administration of liposome formulation

Tissue distribution after administration of the liposome formulation of Example 5 (drug: telavancin) and the liposome formulations of Examples 1, 7, and 8 (drug: oritavancin) were examined by the following methods.

(Tissue distribution test)

The compound of the present invention was intravenously administered at a dose of 5 mg/kg to ICR mice. At a predetermined time, the mice were killed by exsanguination through whole blood collection from the inferior vena cava or the inferior vena cava under isoflurane anesthesia. Then, the liver, kidney, and lung were removed, and a 25% homogenate was prepared with distilled water. The obtained blood was centrifuged, and plasma was then obtained. Thereafter, each sample was measured using LC/MS/MS. The concentration value ratio (each tissue/plasma) obtained was used as a tissue Kp value.

(Experimental results)

Changes in plasma concentration and tissue concentrations in the liver, kidney, and lung are shown in Tables 11 to 22. In the case of telavancin, as shown in Tables 11 and 13, administration of Example 5, which is a liposome formulation, resulted in a higher tissue concentration in the liver, as compared with administration of the drug substance alone, but the tissue Kp value was almost the same. In addition, as shown in Tables 12 and 14, the tissue concentration in the kidney was decreased by administration of Example 5, which is a liposome formulation, as compared with administration of the drug substance alone, and the tissue Kp value was also clearly decreased. Therefore, reduction of side

effects to the kidney was suggested by the liposome formulation.

In the case of oritavancin, as shown in Tables 15, 19, and 21, administration of Examples 7 and 8, which are liposome formulations, resulted in a higher tissue concentration in the liver, and a higher tissue Kp value except for the value after 24 hours, as compared with administration of the drug substance alone. In addition, as shown in Tables 16, 20, and 22, the tissue concentration in the kidney was decreased by administration of Examples 7 and 8, which are liposome formulations, as compared with administration of the drug substance alone, but the tissue Kp value was almost the same. On the other hand, as compared with administration of the drug substance alone, administration of Example 1, which is a liposome formulation, resulted in almost the same tissue concentration in the liver and a decreased tissue Kp value, as shown in Tables 15 and 17. In addition, as shown in Tables 16 and 18, the tissue concentration in the kidney was decreased by administration of Example 1, which is a liposome formulation, as compared with administration of the drug substance alone, and the tissue Kp value was also decreased. Therefore, reduction of side effects to the kidney was suggested by the liposome formulation.

Concentration in plasma and tissue concentration in liver after administration of telavancin drug substance

[Table 11]

| Time after administration (hour) | Plasma | Liver | |
|---|---|---|---|
| | Concentration (ng/mL) | Concentration (ng/mL) | Kp |
| 0.5 | 18200 ± 627 | 3780 ± 201 | 0.21 ± 0.01 |
| 2 | 7700 ± 977 | 4050 ± 256 | 0.53 ± 0.05 |
| 24 | 51.9 ± 89.9 | 2340 ± 465 | 17.16 |

Tissue concentration in kidney and lung after administration of telavancin drug substance

[Table 12]

| Time after administration (hour) | Kidney | | Lung | |
|---|---|---|---|---|
| | Concentration (ng/mL) | Kp | Concentration (ng/mL) | Kp |
| 0.5 | 19000 ± 4110 | 1.05 ± 0.23 | 5860 ± 208 | 0.32 ± 0.03 |
| 2 | 12700 ± 2470 | 1.64 ± 0.12 | 2820 ± 162 | 0.37 ± 0.04 |
| 24 | 5930 ± 1600 | 49.93 | 736 ± 158 | 5.75 |

Concentration in plasma and liver after administration of formulation of Example 5 (drug: telavancin)

[Table 13]

| Time after administration (hour) | Plasma | Liver | |
|---|---|---|---|
| | Concentration (ng/mL) | Concentration (ng/mL) | Kp |
| 0.5 | 42600 ± 7170 | 8590 ± 1550 | 0.21 ± 0.06 |
| 2 | 29100 ± 637 | 11100 ± 2240 | 0.38 ± 0.08 |
| 24 | 6140 ± 554 | 13900 ± 1330 | 2.26 ± 0.08 |

Concentration in kidney and lung after administration of formulation of Example 5 (drug: telavancin)

[Table 14]

| Time after administration (hour) | Kidney | | Lung | |
|---|---|---|---|---|
| | Concentration (ng/mL) | Kp | Concentration (ng/mL) | Kp |
| 0.5 | 7070 ± 1140 | 0.17 ± 0.05 | 6550 ± 1760 | 0.16 ± 0.06 |
| 2 | 5930 ± 1080 | 0.20 ± 0.04 | 3930 ± 719 | 0.14 ± 0.03 |
| 24 | 3560 ± 675 | 0.58 ± 0.09 | 1690 ± 219 | 0.28 ± 0.02 |

Concentration in plasma and liver after administration of oritavancin drug substance

[Table 15]

| Time after administration (hour) | Plasma | Liver | |
|---|---|---|---|
| | Concentration (ng/mL) | Concentration (ng/mL) | Kp |
| 0.5 | 34400 ± 4550 | 14800 ± 1230 | 0.44 ± 0.08 |
| 2 | 14900 ± 1710 | 24900 ± 1400 | 1.68 ± 0.10 |
| 24 | 63.9 ± 12.0 | 38500 ± 2780 | 611.35 ±72.9 |

Concentration in kidney and lung after administration of oritavancin drug substance

[Table 16]

| Time after administration (hour) | Kidney | | Lung | |
|---|---|---|---|---|
| | Concentration (ng/mL) | Kp | Concentration (ng/mL) | Kp |
| 0.5 | 10300 ± 1090 | 0.30 ± 0.04 | 15100 ±563 | 0.44 ± 0.07 |
| 2 | 8720 ± 538 | 0.59 ± 0.05 | 10100 ± 1010 | 0.68 ± 0.09 |
| 24 | 4090 ± 462 | 66.12 ± 17.05 | 3790 ± 388 | 61.19 ± 15.12 |

Concentration in plasma and liver after administration of formulation of Example 1 (drug: oritavancin)

[Table 17]

| Time after administration (hour) | Plasma | Liver | |
|---|---|---|---|
| | Concentration (ng/mL) | Concentration (ng/mL) | Kp |
| 0.5 | 52700 ± 4150 | 10800 ± 685 | 0.20 ± 0.01 |
| 2 | 37400 ± 3730 | 19000 ± 953 | 0.51 ± 0.03 |
| 24 | 5100 ± 1430 | 38700 ± 9190 | 7.95 ± 2.49 |

Concentration in kidney and lung after administration of formulation of Example 1 (drug: oritavancin)

[Table 18]

| Time after administration (hour) | Kidney | | Lung | |
|---|---|---|---|---|
| | Concentration (ng/mL) | Kp | Concentration (ng/mL) | Kp |
| 0.5 | 7380 ± 708 | 0.14 ± 0.01 | 10900 ± 238 | 0.21 ± 0.01 |
| 2 | 6830 ± 842 | 0.18 ± 0.03 | 7060 ± 298 | 0.19 ± 0.02 |
| 24 | 4120 ± 331 | 0.85 ± 0.22 | 3140 ± 215 | 0.65 ± 0.17 |

Concentration in plasma and liver after administration of formulation of Example 7 (drug: oritavancin)

[Table 19]

| Time after administration (hour) | Plasma | Liver | |
|---|---|---|---|
| | Concentration (ng/mL) | Concentration (ng/mL) | Kp |
| 0.5 | 26200 ± 6000 | 47600 ± 9350 | 1.93 ± 0.74 |
| 2 | 6960 ± 740 | 46100 ± 4730 | 6.64 ± 0.58 |
| 24 | 40.6 ± 35.5 | 48900 ± 5210 | 843.63 |

Concentration in kidney and lung after administration of formulation of Example 7 (drug: oritavancin)

[Table 20]

| Time after administration (hour) | Kidney | | Lung | |
|---|---|---|---|---|
| | Concentration (ng/mL) | Kp | Concentration (ng/mL) | Kp |
| 0.5 | 5730 ± 558 | 0.22 ± 0.03 | 8390 ± 1000 | 0.33 ± 0.05 |
| 2 | 3630 ± 300 | 0.53 ± 0.10 | 4380 ± 174 | 0.63 ± 0.05 |
| 24 | 2090 ± 330 | 37.59 | 1830 ± 281 | 31.99 |

Concentration in plasma and liver after administration of formulation of Example 8 (drug: oritavancin)

[Table 21]

| Time after administration (hour) | Plasma | Liver | |
|---|---|---|---|
| | Concentration (ng/mL) | Concentration (ng/mL) | Kp |
| 0.5 | 33700 ± 11300 | 32400 ± 1970 | 1.03 ± 0.34 |
| 2 | 11000 ± 1130 | 51500 ± 2170 | 4.74 ± 0.67 |
| 24 | 73.6 ± 9.0 | 59100 ± 6540 | 804.09 ± 32.46 |

Concentration in kidney and lung after administration of formulation of Example 8 (drug: oritavancin)

[Table 22]

| Time after administration (hour) | Kidney | | Lung | |
|---|---|---|---|---|
| | Concentration (ng/mL) | Kp | Concentration (ng/mL) | Kp |
| 0.5 | 7220 ± 228 | 0.23 ± 0.06 | 9560 ± 689 | 0.30 ± 0.09 |
| 2 | 5880 ± 118 | 0.54 ± 0.05 | 6700 ± 337 | 0.62 ± 0.09 |
| 24 | 2860 ± 324 | 38.96 ± 3.74 | 2330 ± 483 | 31.39 ± 2.63 |

(4) In vitro antibacterial activity after administration of liposome formulation

In vitro antibacterial activity of the liposome formulations of Examples 2 and 3 (drug: compound A), Examples 4 and 5 (drug: telavancin), and the liposome formulations of Examples 1 and 6 to 8 (drug: oritavancin) was examined by the following methods.

(Test methods)

A minimum inhibitory concentration was measured as in vitro antibacterial activity. The minimum inhibitory concentration (MIC: pg/ml) was measured according to the standard method of Japanese Society of Chemotherapy, by an agar plate dilution method using 1000 cfu/spot as an inoculum amount and a susceptibility disk medium as a test medium.

(Experimental results)

The experimental results are shown in Tables 23 to 28. In the tables, a unit of a numerical value of inhibitory activity is pg/ml. In addition, in the tables, S. aureus represents Staphylococcus aureus and E. faecalis represents Gram-positive group D streptococci (enterococci) that are resident in a normal bacterial flora of a human intestinal tract, and can be causative bacteria of endocarditis and the like after surgery. As a result, the liposome formulations of Examples 2 to 8 tended to have a much higher MIC in some of the bacterial species and strains, as compared with the drug substance. On the other hand, it was found that the liposome formulation of Example 1 does not have a much higher MIC and does not have much decreased antibacterial activity as compared with the drug substance for any of the bacterial species including vancomycin-resistant bacteria.

[Table 23]

| Bacterial species | Name of strain | Compound A | | |
|---|---|---|---|---|
| | | Drug substance | Liposome | |
| | | | Example 2 | Example 3 |
| S. aureus | ATCC25923 | 1 | 0.5 | 0.5 |
| S. aureus | SR3637 | 1 | 0.5 | 0.5 |
| S. aureus | ATCC700787 | 2 | > 64 | 16 |
| S. aureus | VRS 1 | 4 | > 64 | > 64 |
| E. faecalis | SR1004 | 0.5 | 0.125 | ≤ 0.063 |
| E. faecalis | SR7914 | 2 | > 64 | > 64 |
| E. faecium | SR7940 | 2 | 64 | 4 |
| E. faecium | SRM1101 | 2 | > 64 | > 64 |

[Table 24]

| Bacterial species | Name of strain | Telavancin | |
|---|---|---|---|
| | | Drug substance | Liposome |
| | | | Example 4 |
| S. aureus | ATCC25923 | 0.5 | 4 |
| S. aureus | SR3637 | 1 | 4 |
| S. aureus | ATCC700787 | 1 | 4 |
| S. aureus | VRS1 | 4 | > 64 |
| E. faecalis | SR1004 | 1 | 2 |
| E. faecalis | SR7914 | 4 | 64 |
| E. faecium | SR7940 | 4 | 16 |
| E. faecium | SRM1101 | 4 | > 64 |

[Table 25]

| Bacterial species | Name of strain | Telavancin | |
|---|---|---|---|
| | | Drug substance | Liposome formulation |
| | | | Example 5 |
| S. aureus | ATCC25923 | 0.25 | 2 |
| S. aureus | SR3637 | 0.25 | 2 |
| S. aureus | ATCC700787 | 1 | 4 |
| S. aureus | VRS 1 | 2 | 8 |
| E. faecalis | SR1004 | 0.5 | 2 |
| E. faecalis | SR7914 | 2 | 16 |
| E. faecium | SR7940 | 2 | > 16 |
| E. faecium | SRM1101 | 4 | 16 |
| | | | |

[Table 26]

| Bacterial species | Name of strain | Oritavancin | |
|---|---|---|---|
| | | Drug substance | Liposome formulation |
| | | | Example 6 |
| S. aureus | ATCC25923 | 4 | 4 |
| S. aureus | SR3637 | 2 | >8 |
| S. aureus | ATCC700787 | 4 | >8 |
| S. aureus | VRS1 | 4 | >8 |
| E. faecalis | SR1004 | 2 | 2 |
| E. faecalis | SR7914 | 2 | 2 |
| E. faecium | SR7940 | 2 | 2 |
| E. faecium | SRM1101 | 2 | 2 |

[Table 27]

| Bacterial species | Name of strain | Oritavancin | |
|---|---|---|---|
| | | Drug substance | Liposome formulation |
| | | | Example 7 |
| S. aureus | ATCC25923 | 4 | 4 |
| S. aureus | SR3637 | 4 | 16 |
| S. aureus | ATCC700787 | 4 | > 64 |
| S. aureus | VRS1 | 4 | 16 |
| E. faecalis | SR1004 | 2 | 2 |
| E. faecalis | SR7914 | 2 | 2 |
| E. faecium | SR7940 | 2 | 2 |
| E. faecium | SRM1101 | 4 | 4 |

[Table 28]

| Bacterial species | Name of strain | Oritavancin | | |
|---|---|---|---|---|
| | | Drug substance | Liposome formulation | |
| | | | Example 1 | Example 8 |
| S. aureus | ATCC25923 | 1 | 2 | 4 |
| S. aureus | SR3637 | 1 | 2 | 4 |
| S. aureus | ATCC700787 | 2 | 4 | > 32 |
| S. aureus | VRS 1 | 2 | 4 | 8 |
| E. faecalis | SR1004 | 1 | 2 | 2 |
| E. faecalis | SR7914 | 1 | 2 | 2 |
| E. faecium | SR7940 | 2 | 2 | 1 |
| E. faecium | SRM1101 | 2 | 4 | 2 |

(5) Toxicity evaluation of liposome formulation

(5-1: In vitro cytotoxicity test)

Cytotoxicity of the compound A drug substance and the liposome formulation of Example 2 was performed by the following methods.

(Test methods)

Vero cells (final concentration: 2.0 × 10 to the power of 4 cells/well) cultured in advance and each test substance were added to 96 wells, and cultured for 72 hours under conditions of 5% $CO_2$ and 37°C. Thereafter, 10 μl/well of a 0.01% resazurin solution was added, and after mixing, the cells were cultured under conditions of 5% $CO_2$ and 37°C for 4 hours, and then a fluorescence value (Ex 531 nm/Em 590 nm) was measured using a microplate reader. A compound concentration (CC50) at which cell viability was 50% was calculated from the fluorescence value at each test substance concentration.

(Results)

The results are shown in Table 29. Liposome formulation resulted in an about 15-fold higher CC50 value than that of the drug substance and reduced toxicity.

[Table 29]

| Test substance | CC50 (pg/ml) |
|---|---|
| Compound A drug substance | 128 |
| Example 2 | 1787 |

(5-2: In vitro hemolysis test)

Hemolysis rates of the compound A drug substance and the liposome formulation of Example 2 were evaluated by the following methods.

(Test methods)

A 10 mM concentration solution of each test substance was prepared using a 5% sugar solution as a solvent, and 12 types of formulation solutions were prepared for each test substance up to 0.0049 mM by 2-fold serial dilution. Using 20% FBS-supplemented M-199 medium (With Earle's salts, sodium bicarbonate and 25 mM HEPES, supplement with 0.1 g/L L-glutamine) as a medium, a 2% red blood cell suspension (defibrated sheep blood) was prepared and mixed with a solvent or each test substance formulation solution (n = 2). In order to use a 100% hemolysis state as a reference of the hemolysis rate, red blood cells were mixed with distilled water at a dilution ratio in preparing the 2% red blood cell suspension to prepare a 100% hemolysis solution. A medium was used in place of the red blood cell suspension, and mixed with each test substance formulation solution (n = 2). A mixed solution of the solvent or each test substance formulation solution and the red blood cell suspension or the medium was incubated for 24 hours, then the supernatant was collected, and an absorbance at 540 nm was measured. At the stage of completion of each incubation, an absorbance of the solvent and the red blood cell suspension, and an absorbance of the solvent and the medium were set to a reference of "hemolysis rate 0%", and an absorbance of the 100% hemolysis solution was set to a reference of "100% hemolysis", and a hemolysis rate by mixing and incubating each test substance formulation solution and the red blood cell suspension was calculated by the following calculation formula.

$$PH\ (\%) = (CR \cdot VM) \times 100/(VL \cdot VR)$$

PH is a hemolysis rate, CR is an absorbance when the test substance adjustment solution and the 2% red blood cell suspension are mixed, VM is an absorbance when the test substance adjustment solution and the medium are mixed, VL is an absorbance of the 100% hemolysis solution, and VR is an absorbance when a DMSO solution and the 2% red blood cell suspension are mixed.

(Results)

The results are shown in Table 30. By making it into a liposome formulation, hemolytic activity was no longer confirmed.

[Table 30]

| Test substance | Hemolysis rate (%) |
|---|---|
| Compound A drug substance | 34 |
| Example 2 | 1 |

[INDUSTRIAL APPLICABILITY]

The liposome formulation of the present invention is a liposome formulation in which an antibacterial agent is encapsulated in a liposome, a lipid-soluble side chain of the antibacterial agent is bound to a lipid of the liposome, and the antibacterial agent extends outward from a surface of the liposome. By making such a configuration of formulation, a liposome formulation having less side effects and high antibacterial activity can be produced.

## Claims

1. A liposome formulation, wherein an antibacterial agent is bound outward to a surface layer of a liposome.

2. The liposome formulation according to claim 1, wherein a lipid-soluble side chain of the antibacterial agent is bound to a lipid of the liposome.

3. The liposome formulation according to claim 2, wherein the lipid-soluble side chain of the antibacterial agent is a group represented by the following formulas:

[Chemical Formula 1]

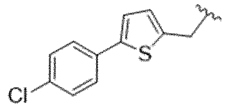
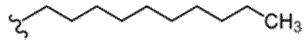
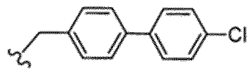
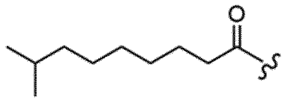
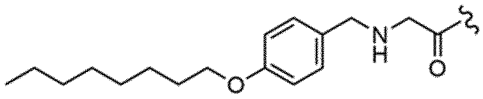
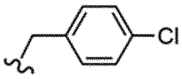
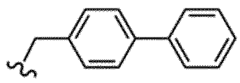
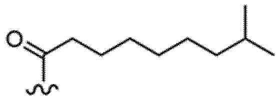
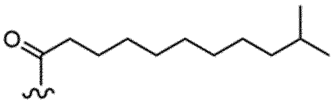
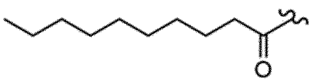
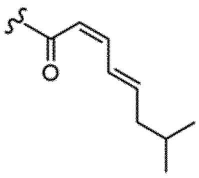
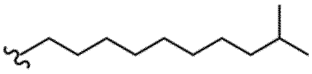
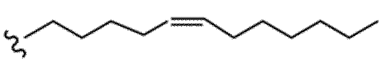
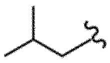
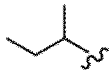
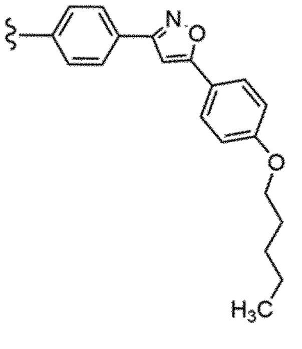
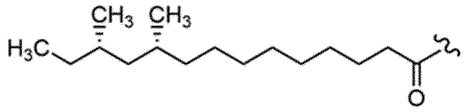

[Chemical Formula 2]

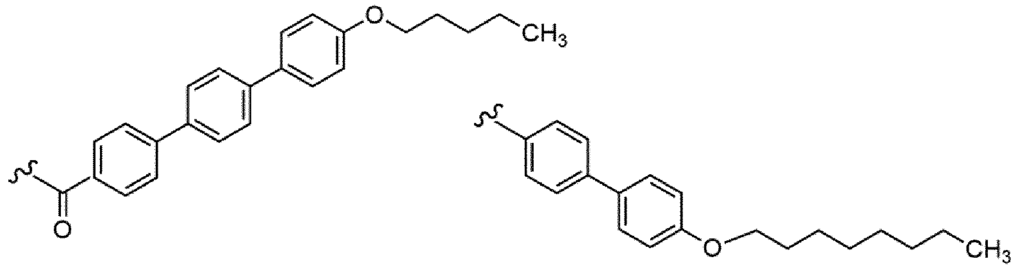

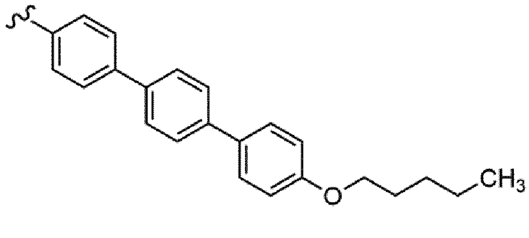

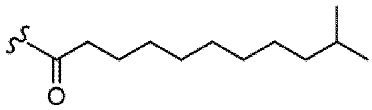

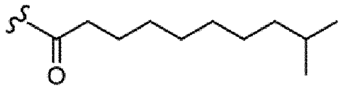

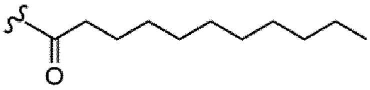

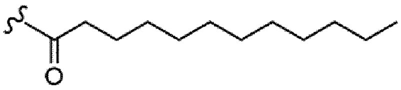

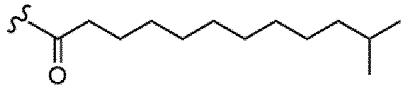

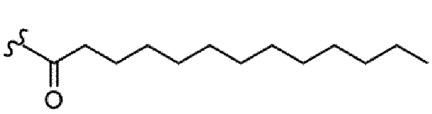

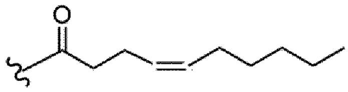

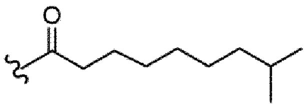

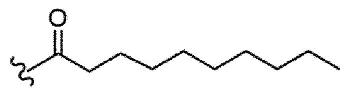

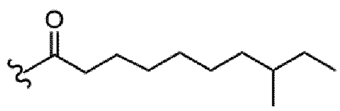

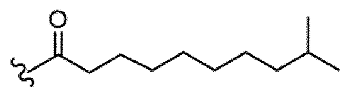

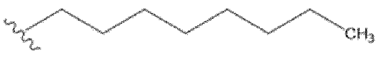

.

4. The liposome formulation according to any one of claims 1 to 3, wherein the antibacterial agent is a glycopeptide antibacterial agent or a cyclic lipopeptide antibacterial agent.

5. The liposome formulation according to claim 1 or 2, wherein the antibacterial agent is one or more selected from the group consisting of a compound represented by Formula (I):

[Chemical Formula 3]

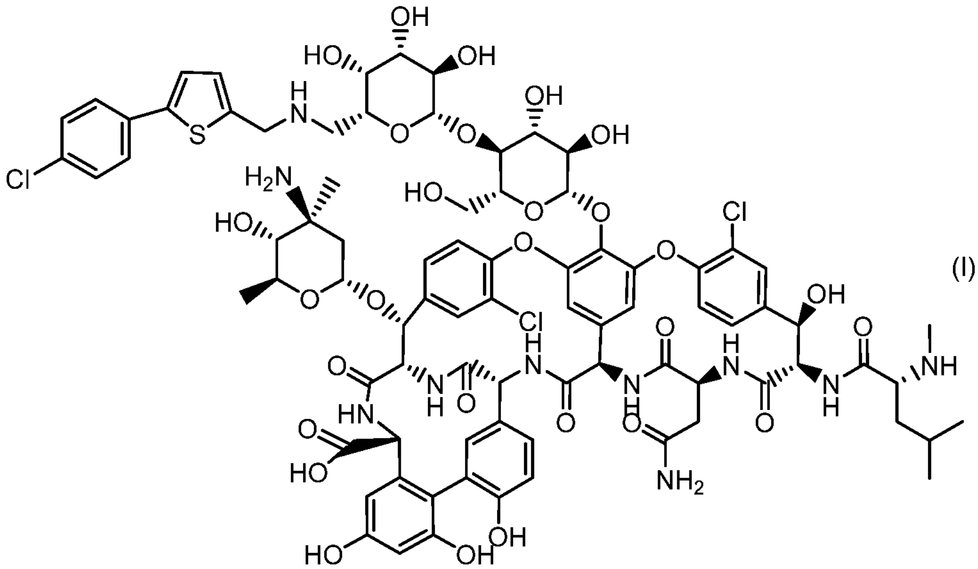

(I)

or a pharmaceutically acceptable salt thereof, telavancin, oritavancin, teicoplanin, dalbavancin, mideplanin, N'-p-octyloxybenzylglycyl-vancomycin, (4"R)-22-O-(3-amino-3-C-methyl-2,3,6-trideoxy-α-L-arabino-hexopyranosyl)-3"-[(4-chlorobenzyl)amino]-3"-deaminovancomycin, (4"R)-3"-N-(1, 1'-biphenyl-4-ylmethyl)-22-O-(3-amino-3-C-methyl-2,3,6-trideoxy-α-L-arabino-hexopyranosyl)vancomycin, and 5,31-dichloro-38-de(methoxycarbonyl)-7-demethyl-15-deamino-19-deoxy-56-O-[2-deoxy-2-[(10-methyl-1-oxoundecyl)amino]-5-D-glucopyranosyl]-38-[[[3-(dimethylamino)propyl]amino]carbonyl]-42-O-α-D-mannopyranosyl-15-(methylamino)ristomycin A aglycone, and YV11455.

6. The liposome formulation according to claim 1 or 2, wherein the antibacterial agent is oritavancin.

7. The liposome formulation according to any one of claims 1 to 6, wherein the liposome is comprised of one or two or more lipids.

8. The liposome formulation according to claim 7, wherein the lipid constituting the liposome is one or more selected from the group consisting of a phospholipid, an ether glycerophospholipid, a sphingophospholipid, a glyceroglycolipid, a glycosphingolipid, a glyceride lipid, cardiolipin, a galactolipid, a mannolipid, galactolecithin, and cholesterol.

9. The liposome formulation according to claim 7, wherein the lipid constituting the liposome is one or more selected from the group consisting of egg yolk lecithin, soybean lecithin, hydrogenated egg yolk lecithin, hydrogenated soybean lecithin, a hydrogenated soybean phospholipid (HSPC), soybean-derived phosphatidylcholine, soybean-derived hydrogenated phosphatidylcholine, a purified hydrogenated soybean phospholipid, phosphatidylcholine, egg yolk phosphatidylcholine, hydrogenated soybean phosphatidylcholine, dimyrisylphosphatidylcholine, dipalmitoylphosphatidylcholine, palmitoylstearoylphosphatidylcholine, dioleoylphosphatidylcholine, distearoylphosphatidylcholine, 1,2-dioleoyl-sn-glycero-3-phosphatidylcholine, distearylphosphatidylcholine, 1-palmitoyl-2-oleoyl-sn-glycero-3-phosphatidylcholine, phosphatidylethanolamine, soybean phosphatidylethanolamine, dimyristoylphosphatidylethanolamine, dipalmitoylphosphatidylethanolamine, dioleoylphosphatidylethanolamine, egg yolk phosphatidylethanolamine, monooleoylphosphatidylethanolamine, polyethylene glycol-distearoylphosphatidylethanolamine, phosphatidylglycerol, soybean phosphatidyl glycerol, dimyristoylphosphatidylglycerol, dipalmitoylphosphatidyl glycerol, dioleoylphosphatidyl glycerol, distearylphosphatidylglycerol, dielaidoylphosphatidylglycerol, palmitoylstearoylphosphatidylglycerol, egg yolk phosphatidylglycerol, phosphatidylserine, egg yolk phosphatidylserine, soybean phosphatidylserine, dimyristoylphosphatidylserine, dipalmitoylphosphatidylserine, dioleoylphosphatidylserine, dielaidoylphosphatidylserine, 1,2-dioleoyl-sn-glycero-3-phosphatidylserine, distearoylphosphatidylserine, phosphatidic acid, egg yolk phosphatidic acid, soybean phosphatidic acid, dimyristoylphosphatidic acid, dipalmitoylphosphatidic acid, dioleoylphosphatidic acid, distearylphosphatidic acid, dielaidoylphosphatidylamine, soybean-derived hydrogenated phosphatidylinositol, egg yolk phosphatidylinositol, soybean phosphatidylinositol, dipalmitoylphosphatidylinositol, 1,2-dioleoyl-sn-glycero-3-phosphatidylinositol, dimyristoylphosphatidylinositol, distearoylphosphatidylinositol, cholesterol, cardiolipin, sphingomyelin, hexadecylphosphocholine, N-[1-(2,3-dioleyloxy)propyl]-N,N,N-trimethylammonium chloride, 1,2-bis(oleoyloxy)-3-(trimethylammonio)propane, 1,2-bis(hexadecyloxy)-3-trimethylaminopropane, 3[beta][N-(N'N'-dimethylaminoethane)-carbamyl] cholesterol, 1,2-dimyristroyl-sn-glycero-3-phosphocholine, 1,2-dilauroyl-sn-glycero-3-phosphocholine, 1,2-distearoyl-sn-glycero-3-phosphocholine, 1,2-dimyristoyl-sn-glycero-3-phosphoethanolamine, 1,2-dipalmitoyl-sn-glycero-3-phosphoethanolamine, 1,2-dioleoyl-sn-glycero-3-phosphate monosodium salt, 1,2-dipalmitoyl-sn-glycero-3-[phosphor-rac-(l-glycerol)] sodium salt, 1,2-dimyristoyl-sn-glycero-3-[phospho-L-serine] sodium salt, 1,2-dioleoyl-sn-glycero-3-phosphoethanolamine-N-glutaryl sodium salt, 1,1',2,2'-tetramyristoyl cardiolipin ammonium salt, a glyceride lipid, cardiolipin, a galactolipid, a mannolipid, and galactolecithin.

**10.** The liposome formulation according to claim 7, wherein the lipid constituting the liposome is a hydrogenated soybean phospholipid (HSPC) and/or cholesterol.

**11.** The liposome formulation according to claim 1 or 2, wherein the antibacterial agent is oritavancin and the lipid constituting the liposome is a hydrogenated soybean phospholipid (HSPC) and cholesterol.

**12.** The liposome formulation according to any one of claims 1 to 11, wherein a surface charge of the liposome is a positive charge.

**13.** The liposome formulation according to any one of claims 1 to 12, wherein an average particle size of the liposome is 30 to 120 nm.

**14.** The liposome formulation according to any one of claims 1 to 13, wherein a weight ratio of the lipid constituting the liposome to the antibacterial agent in the liposome is 15 : 1 to 20 : 1.

**15.** A method for producing a liposome formulation in which an antibacterial agent is bound outward to a surface layer of a liposome, the method comprising the steps of:

i) mixing a first solution in which one or more lipids are dissolved in a first organic solvent and a second solution in which an antibacterial agent is dissolved in a second organic solvent to obtain a third solution;
ii) providing one or more first flows of the third solution, a feed rate of which is a linear velocity of 1.0 m/min or more;
iii) providing one or more second flows of a first aqueous solution, a feed rate of which is a linear velocity of 1.1 m/min or more;
iv) combining the one or more first flows and the one or more second flows at a first crossing point to mix components of the first and second flows and providing a first combined flow containing the antibacterial agent-containing liposome; and
v) in a first direction, flowing the first combined flow and providing an outlet solution containing the antibacterial agent-containing liposome.

**16.** The production method according to claim 15, wherein the first and second organic solvents are miscible with the first aqueous solution, either the first and second organic solvents or the first aqueous solution contains a buffer, and a concentration of the first organic solvent in the outlet solution is less than 45%.

**17.** The production method according to claim 15 or 16, wherein the first organic solvent contains a lower alkanol solvent, the second organic solvent contains an aprotic polar solvent, and the buffer is an aqueous solution containing a basic peptide and/or an alkali metal salt.

**18.** The production method according to claim 17, wherein the lower alkanol solvent comprises ethanol, the aprotic polar solvent comprises dimethyl sulfoxide, and the buffer comprises histidine and/or sodium phosphate.

**19.** The production method according to any one of claims 15 to 18, further comprising a step of incubating the outlet solution.

**20.** The prouction method according to claim 19, further comprising a step of concentrating and dialyzing the incubated outlet solution.

**21.** The production method according to any one of claims 15 to 20, wherein about 80% or more of the antibacterial agent is encapsulated in a lipid nanoparticle in the outlet solution.

Fig. 1

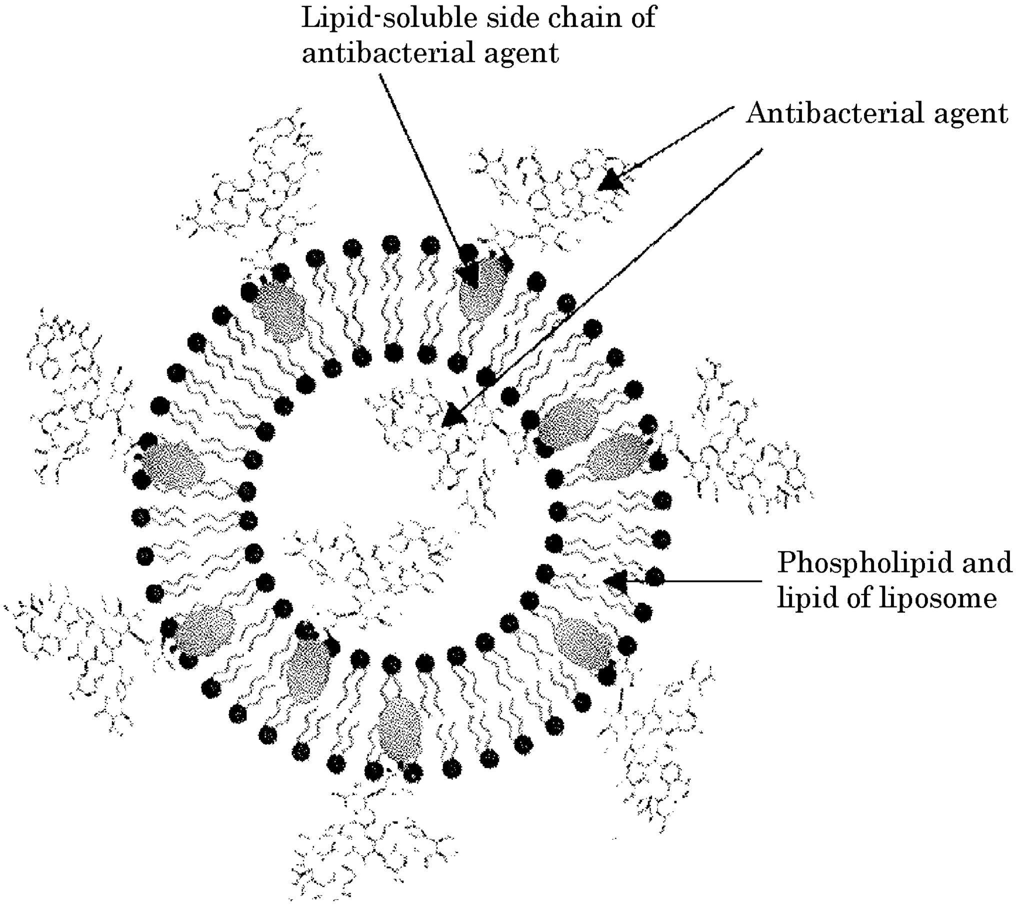
Lipid-soluble side chain of antibacterial agent
Antibacterial agent
Phospholipid and lipid of liposome

Fig. 2

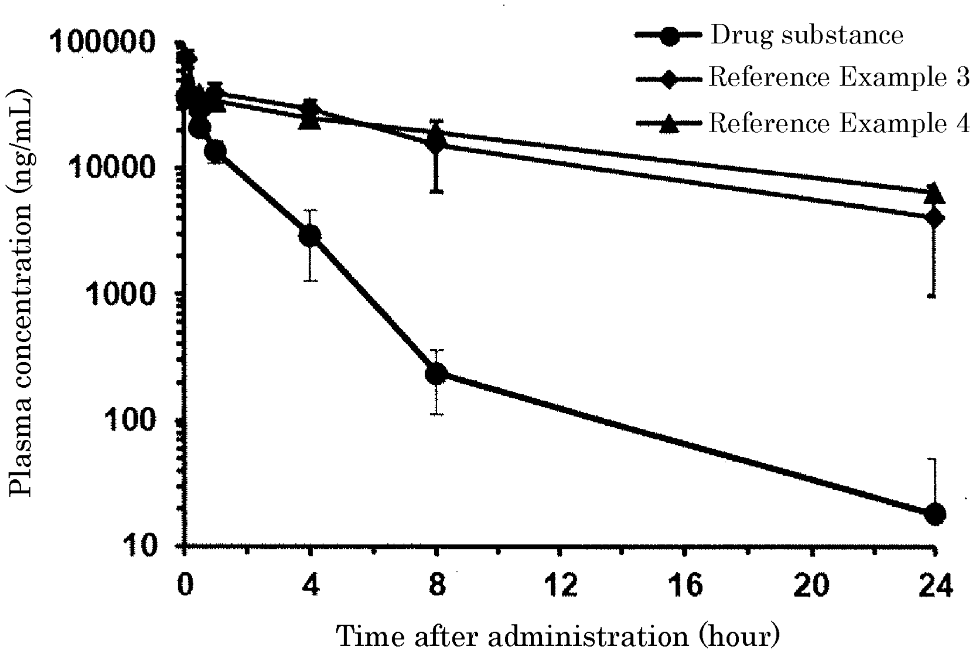
Drug substance
Reference Example 3
Reference Example 4
Plasma concentration (ng/mL)
100000
10000
1000
100
10
0
4
8
12
16
20
24
Time after administration (hour)

Fig. 3

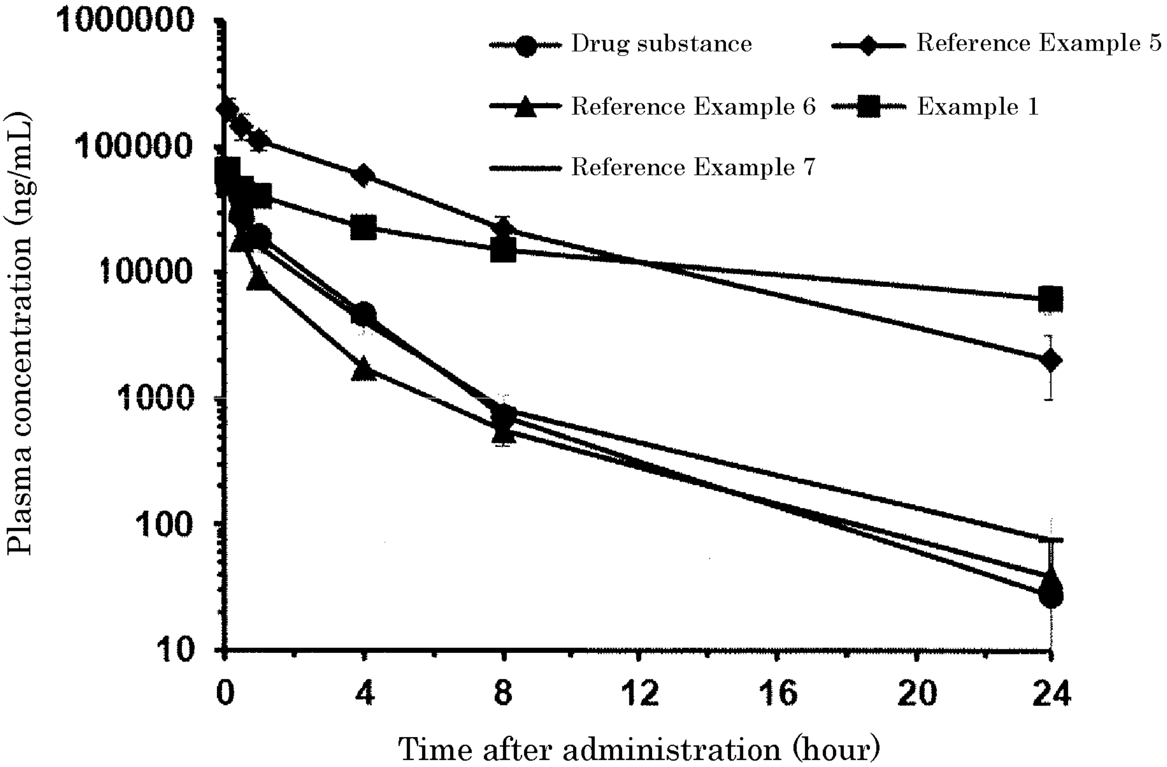
1000000
100000
10000
1000
100
10
Plasma concentration (ng/mL)
0
4
8
12
16
20
24
Time after administration (hour)
Drug substance
Reference Example 5
Reference Example 6
Example 1
Reference Example 7

**INTERNATIONAL SEARCH REPORT**

International application No. **PCT/JP2021/032378**

**A. CLASSIFICATION OF SUBJECT MATTER**

***A61K 47/54***(2017.01)i; ***A61K 9/127***(2006.01)i; ***A61K 45/00***(2006.01)i; ***A61P 31/04***(2006.01)i
FI: A61K47/54; A61K9/127; A61K45/00; A61P31/04

According to International Patent Classification (IPC) or to both national classification and IPC

**B. FIELDS SEARCHED**

Minimum documentation searched (classification system followed by classification symbols)

A61K47/54; A61K9/127; A61K45/00; A61P31/04

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2018-100285 A (VYOME BIOSCIENCES PVT. LTD.) 28 June 2018 (2018-06-28) claims, paragraphs [0063]-[0073], examples | 1-14 |
| X | JP 3-34920 A (STRASSMANN, Gideon) 14 February 1991 (1991-02-14) claims, p. 3, upper right column, line 15 to p. 5, upper right column, line 4, p. 6, lower right column, line 2 to p. 10, lower left column, line 14, examples 5-7 | 1, 7-10, 12-14 |
| X | JP 9-502700 A (THE LIPOSOME CO., INC.) 18 March 1997 (1997-03-18) claims, p. 14, lines 5-17, p. 17, line 12 to p. 18, line 4, p. 24, lines 4-22, examples 17-27 | 1-14 |
| X | JP 2007-502323 A (ALZA CORP.) 08 February 2007 (2007-02-08) claims, paragraphs [0046]-[0052], examples | 1, 7-10, 12-14 |
| X | JP 2016-505545 A (INSMED INC.) 25 February 2016 (2016-02-25) claims, paragraphs [0065]-[0080], [0088]-[0109], [0126], examples 3, 4, fig. 14 | 1-21 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 October 2021** | **19 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/032378**

| Patent document cited in search report | Publication date (day/month/year) | Patent family member(s) | Publication date (day/month/year) |
|---|---|---|---|
| JP 2018-100285 A | 28 June 2018 | US 2014/0364595 A1<br>claims, paragraphs [0084]-[0104], examples<br>WO 2012/177986 A2<br>EP 2723452 A | |
| JP 3-34920 A | 14 February 1991 | EP 393707 A2<br>claims, p. 2, line 17 to p. 3, line 29, p. 4, line 44 to p. 6, line 49, examples 5-7<br>KR 10-1990-0015714 A | |
| JP 9-502700 A | 18 March 1997 | US 5540936 A<br>claims, column 5, lines 24-44, column 7, lines 37-63, column 11, line 34 to column 14, line 29, examples 17-27<br>WO 1994/022430 A1<br>EP 692961 A | |
| JP 2007-502323 A | 08 February 2007 | US 2003/0054028 A1<br>claims, paragraphs [0075]-[0088], examples<br>WO 2004/110497 A2<br>EP 1617873 A | |
| JP 2016-505545 A | 25 February 2016 | US 2015/0314002 A1<br>claims, paragraphs [0044]-[0080], [0088]-[0108], examples 3, 4, fig. 14<br>WO 2014/085526 A1<br>EP 2925298 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008512350 A
- JP 2019514985 A


### Non-patent literature cited in the description


- Drug delivery system: controlled release absorption improvement drug targeting. 124